# EUROPÄISCHE PATENTSCHRIFT

(11) **EP 1 133 477 B1**
(45) Veröffentlichungstag und Bekanntmachung des Hinweises auf die Patenterteilung: **18.02.2004**
(21) Anmeldenummer: 99964497.4
(22) Anmeldetag: 23.11.1999
(51) Int. Cl.: C07D 235/30, C07D 401/04, C07D 401/14, C07D 403/04, A61K 31/4184, A61K 31/454, A61K 31/496, A61P 25/00, A61P 9/00

(54) **SUBSTITUIERTE BENZIMIDAZOLE UND IHRE VERWENDUNG ALS PARP INHIBITOREN**
SUBSTITUTED BENZIMIDAZOLES AND THEIR USE AS PARP INHIBITORS
BENZIMIDAZOLES SUBSTITUES ET LEUR UTILISATION COMME INHIBITEURS DE LA PARP

(30) Priorität: 27.11.1998 DE 19854933; 12.04.1999 DE 19916460
(43) Veröffentlichungstag der Anmeldung: 19.09.2001
(73) Patentinhaber: Abbott GmbH & Co. KG, 65205 Wiesbaden (DE)
(72) Erfinder: LUBISCH, Wilfried, D-69115 Heidelberg (DE); KOCK, Michael, D-67105 Schifferstadt (DE); HÖGER, Thomas, D-68535 Edingen-Neckarhausen (DE); SCHULT, Sabine, D-67346 Speyer (DE); GRANDEL, Roland, D-69221 Dossenheim (DE); MÜLLER, Reinhold, D-67105 Schifferstadt (DE)
(74) Vertreter: Grünecker, Kinkeldey, Stockmair & Schwanhäusser Anwaltssozietät
(86) Internationale Anmeldenummer: PCT/EP1999/009004
(87) Internationale Veröffentlichungsnummer: WO 2000/032579

(56) Entgegenhaltungen:
- WO-A-97/04771
- WO-A-98/33802

## Beschreibung

Die vorliegende Erfindung betrifft neuartige Benzimidazole, ihre Herstellung und die Verwendung als Inhibitoren des Enzyms Poly(ADP-ribose)polymerase oder PARP (EC 2.4.2.30) zur Herstellung von Arzneimitteln.

Poly(ADP-ribose)polymerase (PARP) bzw. wie es auch genannt wird Poly(ADP-ribose)synthase (PARS) stellt ein regulatorisches Enzym dar, das in Zellkernen gefunden wird (K. Ikai et al., *J. Histochem. Cytochem.* 1983, *31*, 1261-1264). Man nimmt an, daß PARP eine Rolle bei der Reparatur von DNA-Brüchen spielt (M.S. Satoh et al., *Nature* 1992, *356,* 356-358). Schädigungen oder Brüche der DNA-Stränge aktivieren das Enzym PARP, das, wenn es aktiviert ist, die Übertragung von ADP-Ribose aus NAD katalysiert (S. Shaw, *Adv. Radiat. Biol*., 1984, *11*, 1-69). Dabei wird Nikotinamid aus NAD freigesetzt. Nikotinamid wird unter Verbrauch des Energieträgers ATP von anderen Enzymen wieder in NAD umgewandelt. Eine Überaktivierung von PARP hätte dementsprechend einen unphysiologisch hohen Verbrauch von ATP zur Folge und dies führt im Extremfall zu Zellschädigungen und Zelltod.

Es ist bekannt, daß Radikale wie Superoxid-Anion, NO und Wasserstoffperoxid in Zellen zu DNA-Schädigungen führen können und damit PARP aktivieren. Die Bildung von großen Mengen an Radikalen wird bei eine Reihe von pathophysiologischen Zuständen beobachtet und geht davon aus, daß diese Anhäufung von Radikalen zu den beobachteten Zell- bzw. Organschäden führen oder beitragen. Dazu zählt von zum Beispiel ischämische Zustände von Organen wie im Schlaganfall, Herzinfarkt ( C. Thiemermann et al., *Proc. Natl. Acad. Sci. USA,* 1997, *94*, 679-683) oder Ischämie der Nieren, aber auch Reperfusionsschäden wie sie zum Beispiel nach der Lyse von Herzinfarkt auftreten (s. oben: C. Thiemermann et al.). Die Hemmung von dem Enzym PARP könnte demzufolge ein Mittel sein, um diese Schäden zum mindestens zum Teil zu verhindern oder abzumildern. PARP-Inhibitoren könnten somit ein neues Therapieprinzip zur Behandlung von eine Reihe von Krankheiten darstellen.

Das Enzym PARP beeinflußt die Reparatur von DNA-Schäden und könnte somit auch in der Therapie von Krebs-Erkrankungen eine Rolle spielen, da in Kombination mit cytostatisch wirksamen Stoffen ein höheres Wirkpotential gegenüber Tumorgewebe beobachtet wurde (G. Chen et al. *Cancer Chemo. Pharmacol.* 1988, *22*, 303).

Nicht limitierende Beispiele für Tumoren sind Leukämie, Glioblastome, Lymphome, Melanome, Mama- und Cervicalkarzinome.

Zudem wurde gefunden, daß PARP-Inhibitoren immunosuppressive Wirkung zeigen können (D. Weltin et al. *Int. J. Immunopharmacol.* 1995, *17*, 265-271).

Es wurde ebenfalls entdeckt, daß PARP bei immunologischen Erkrankungen bzw. Krankheiten, in denen das Immunsystem eine wichtige Rolle spielt, wie zum Beispiel rheumatoide Arthritis und septischer Schock, involviert ist, und daß PARP-Inhibitoren einen günstigen Effekt auf den Krankheitsverlauf zeigen können (H. Kröger et al. *Infammation* 1996, *20*, 203-215; W. Ehrlich et al. *Rheumatol. Int.* 1995, *15*, 171-172; C. Szabo et al., *Proc. Natl. Acad. Sci. USA* 1998, *95*, 3867-3872; S. Cuzzocrea et al. *Eur. J. Pharmacol.* 1998, *342*, 67-76).

Unter PARP im Sinne dieser Erfindung werden auch Isoenzyme des oben beschriebenen PARP-Enzyms verstanden.

Weiterhin zeigte der PARP-Inhibitor 3-Aminobenzamid protektive Effekte in einem Model für den Kreislaufschock (S. Cuzzocrea et al., *Br. J. Pharmacol.* 1997, *121*, 1065-1074).

Außerdem ist PARP bei Diabetes mellitus involviert (V. Burkhart et al., Nature Medicine, 1999, 5314-19).

Benzimidazole sind vielfach beschrieben worden.

Die Synthese von 2-Phenyl-benzimidazyl-4-amiden, die am Amid-Rest noch eine substituierte Alkyl-Kette tragen, und die cytotoxische Wirkung haben sollen, sind in J. Med. Chem. 1990, 33, 814-819 aufgeführt. In WO 97/04771 sind Benzimidazol-4-amide aufgeführt, die das PARS hemmen. Insbesondere sind Derivate dort als wirksam beschrieben , die einen Phenyl-Ring in 2-Stellung tragen, wobei der Phenyl-Ring noch mit einfachen Substituenten wie Nitro, Methoxy und CF₃, substituiert sein kann. Obwohl diese Substanzen zum Teil gute Hemmung des Enzyms PARP zeigen, haben die dort beschrieben Derivate den Nachteil, daß sie nur gering oder keine Löslichkeit in wäßrigen Lösungen zeigen und somit nicht als wäßrige Lösung appliziert werden können.

Benzimidazole, die in 2-Stellung ein Piperidin-Ring tragen, sind ebenfalls bereits beschrieben worden. So sind in J. Het. Chem. 1987, 24, 31 Derivate als Antihistaminika hergestellt worden. In J. Het. Chem. 1995, 32, 707 und J. Het. Chem. 1989, 26, 541 sind analoge Verbindungen mit der gleichen Verwendung beschrieben worden. In EP 818454 sind 2-Piperidinylbenzimidazole als Antihistaminika und in WO 9736554 als Mittel gegen Hepatitis erwähnt. Ebenfalls in CA 80, 146143, Fr. 2103639 und in Khim. Geterotsikl. Soedin 1974, 1, 104, sind Derivate aufgeführt. In WO 98/33802 werden Chinazolinonverbindungen beschrieben, die eine PARP-inhibitierende Wirkung zeigen.

Allerdings ist die Bedeutung von Substituenten am Phenylaromaten im Benzimidazolfragment nicht untersucht worden. Des weiteren sind solche Benzimidazole, die einen 4- bis 8-gliedrigen Heterozyklus, insbesondere einen Piperidin-Ring in 2-Stellung tragen, bisher nicht als PARP-Inhibitoren beschrieben worden.

In der vorliegenden Anmeldung wird nun der überraschende Befund beschrieben, daß, wenn man ein Carbonsäureamid-Rest am Benzimidazol-Aromaten einführt, Benzimidazole erhält, die neuartige und gut wirksame PARP-Inhibitoren darstellen, sofern sie in 2-Stellung mit einem gesättigten Heterozyklus substituiert sind.

In einer Reihe von Therapien wie Schlaganfall werden die Wirkstoffe intravenös als Infusionslösung appliziert. Dazu ist es notwendig Substanzen, hier PARP-Inhibitoren, zur Verfügung zu haben, die ausreichende Wasserlöslichkeit bei physiologischen pH-Werten oder angenäherten pH-Werten (z.B: pH-Werten von 5-8) aufweisen, so daß eine Infusionslösung hergestellt werden kann. Viele der beschriebenen PARP-Inhibitoren, insbesondere die besser wirksamen PARP-Inhibitoren, haben jedoch den Nachteil, daß sie nur geringe oder keine Wasserlöslichkeit bei diesen pH-Werten zeigen und somit nicht für eine intravenöse Applikation in Frage kommen. Derartige Wirkstoffe können nur mit Hilfsstoffen, die die Wasserlöslichkeit vermitteln sollen, appliziert werden (vgl. WO 97/04771 ). Diese Hilfsstoffe, zum Beispiel Polyethylenglykol und Dimethylsulfoxid, verursachen häufig Nebeneffekte oder sind sogar unverträglich. Gut wirksame PARP-Inhibitoren mit ausreichender Wasserlöslichkeit sind bisher nicht beschrieben worden.

Es wurde überraschenderweise gefunden, daß Benzimidazole, die am Imidazol-Ring einen Piperidin-Ring tragen, gut wirksame Inhibitoren darstellen und die durch den Einbau des aliphatischen Amin-Restes eine Salzbildung mit Säuren ermöglichen, das dadurch eine deutlich verbesserte Wasserlöslichkeit zeigen und somit die Herstellung einer Infusionslösung ermöglicht.

In der vorliegenden Erfindung werden neue Benzimidazole-Derivate der allgemeinen Formel I beschrieben, die gegenüber den bereits beschriebenen Verbindungen Vorteile zeigen und potente PARP-Inhibitoren darstellen und zugleich auch ausreichende Wasserlöslichkeit zeigen. Wird von Verbindungen der Formel I gesprochen, werden darunter die Verbindungen der Formel Ia und Ib verstanden.

Gegenstand der vorliegenden Erfindung sind substituierte Benzimidazole der allgemeinen Formel I: 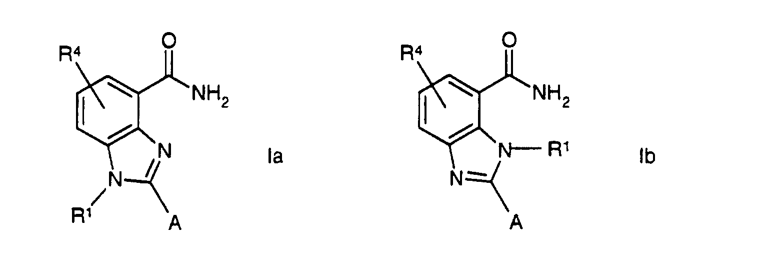 worin
- R¹: Wasserstoff, verzweigtes und unverzweigtes C₁-C₆-Alkyl, wobei ein C-Atom des Alkyl-Restes noch OR⁵ (wobei R⁵ Wasserstoff oder C₁-C₄-Alkyl bedeutet), oder ein C-Atom in der Kette auch eine =O-Gruppe oder eine Gruppe NR⁸R⁹ tragen kann, wobei R⁸ und R⁹ unabhängig voneinander Wasserstoff oder C₁-C₄-Alkyl bedeuten und NR⁸R⁹ zusammen ein zyklisches Amin mit 4 bis 8 Ringatomen sein kann, wobei die C-Ketten in R⁸ bzw. R⁹ oder der durch NR⁸R⁹ gebildete Ring noch einen Rest R⁶ tragen kann, der unabhängig von R² dieselben Bedeutung wie R² annehmen kann und
- R⁴: Wasserstoff, verzweigtes und unverzweigtes C₁-C₆-Alkyl, Chlor, Brom, Fluor, Nitro, Cyano, NR⁸R⁹, NH-CO-R¹⁰, OR⁸, wobei R⁸ und R⁹ unabhängig voneinander Wasserstoff oder C₁-C₄-Alkyl bedeuten und NR⁸R⁹ zusammen ein zyklisches Amin mit 4 bis 8 Ringatomen sein kann, wobei der Ring noch einen Rest (verzweigtes und unverzweigtes C₁-C₆-Alkyl, C₃-C₇-Cycloalk-C₁-C₄-Alkyl, CO-R⁴¹, COOR⁴¹ und Phenyl) tragen kann, und R¹⁰ Wasserstoff, C₁-C₄-Alkyl oder Phenyl bedeuten kann und R⁴¹ dieselben Bedeutungen wie R²¹ annehmen kann,
- A: einen gesättigten oder einfach ungesättigten heterozyklischen, 4- bis 8-gliedrigen Ring, der ein oder zwei Stickstoff-Atome enthält, wobei zusätzlich noch ein Sauerstoff- oder Schwefel-Atom eingebaut sein kann, der durch die Substituenten R² und R³ substituiert ist, wobei
- R²: Wasserstoff, verzweigtes und unverzweigtes C₁-C₈-Alkyl, das noch mit R²³ substituiert sein kann und ein C-Atom der Kette eine =O-Gruppe tragen kann, C₃-C-₇-Cycloalk-C₁-C₄-Alkyl, -CO-(NH)_{0,1}-R²¹, COOR²¹ und Phenyl bedeuten kann, wobei R²¹ Wasserstoff, verzweigtes und unverzweigtes C₁-C₆-Alkyl, C₃-C₇-Cycloalk-C₁-C₄-Alkyl, Phen-C₁-C₄-Alkyl, C₃-C₇-Cycloalkyl und Phenyl bedeuten kann und jeder Rest noch (CH₂)₀₋₂-R²³ tragen kann, und der jeweilige Phenylring seinerseits noch tragen kann, und der jeweilige Phenylring seinerseits noch mit 1, 2 oder 3 der folgenden Resten substituiert sein kann: Chlor, Fluor, Brom, Jod, verzweigtes und unverzweigtes C₁-C₄-Alkyl, Nitro, CF₃, Cyano, -(CH₂)₀₋₂-NR²⁴R²⁵, NH-CO-R¹⁰, OR¹⁰, COOR¹⁰, SO₂-C₁-C₄-Alkyl, SO₂Ph, SO₂NH, NHSO₂-C1-C₄-Alkyl, NHSO₂Ph und CF₃, wobei R²⁴ und R²⁵ unabhängig voneinander Wasserstoff oder C₁-C₄-Alkyl bedeuten und NR²⁴R²⁵ zusammen ein zyklisches Amin mit 4 bis 8 Ringatomen sein kann, wobei der Ring noch ein Rest verzweigtes und unverzweigtes C₁-C₆-Alkyl, C₃-C₇-Cycloalk-C₁-C₄-Alkyl, CO-R²², COOR²² (mit R²² gleich Wasserstoff, verzweigtes oder unverzweigtes C₁-C₆-Alkyl, C₃-C₇-Cycloalk-C₁-C₄-Alkyl, Phen-C₁-C₄-Alkyl, C₃-C₇-Cycloalkyl und Phenyl) und Phenyl tragen kann, und R¹⁰ Wasserstoff, C₁-C₄-Alkyl oder Phenyl bedeuten, und
- R²³: NR²⁶R²⁷ bedeutet, wobei R²⁶ und R²⁷ Wasserstoff, C₁-C₆-Alkyl, C₀-C₄-Alkyl-Phenyl, wobei der Phenylring noch mit bis zu 3 Resten Cl, F, Br, J, C₁-C₄-Alkyl, CF₃, CN, SO₂-C₁-C₄-Alkyl, SO₂-Phenyl, NO₂, NH₂, NHCO-C₁-C₄-Alkyl, NHCO-Phenyl, OH, O-C₁-C₄-Alkyl, O-C₁-C₄-Alkyl-Phenyl substituiert sein kann, und NR²⁶R²⁷ auch ein cyclisches Amin mit 3 bis 8 Gliedern darstellen kann, wobei zusätzlich noch ein weiteres Hetero-atom wie O, N und S enthalten sein kann und der Ring noch mit einem Rest R²⁸ substituiert sein kann, wobei R²⁸ C₁-C₄-Alkyl und C₁-C₄-Alkyl-Phenyl sein kann,
- R³: Wasserstoff, verzweigtes und unverzweigtes C₁-C₈-Alkyl, gegebenenfalls durch C₁-C₆-Alkyl substituiertes C₃-C₇-Cyclo-alk-C₁-C₄-Alkyl, gegebenenfalls durch C₁-C₆-Alkyl substituiertes C₃-C₇-Cycloalkyl, wobei ein C-Atom des Restes noch einen Phenyl-Ring tragen kann, der seinerseits noch mit 1, 2 oder 3 der folgenden Resten substituiert sein kann: Chlor, Fluor, Brom, Jod, verzweigtes und unverzweigtes C₁-C₄-Alkyl, Nitro, Cyano, (CH₂)₀₋₂-NR³²R³³, NH-CO-R¹⁰, OR¹⁰, COOR¹⁰, SO₂-C₁-C₄-Alkyl, SO₂Ph, SO₂NH₂, NHSO₂-C₁-C₄-Alkyl, NHSO₂Ph und CF₃, wobei R³² und R³³ unabhängig voneinander Wasserstoff oder C₁-C₄-Alkyl bedeuten und NR³²R³³ zusammen ein zyklisches Amin mit 4 bis 8 Ringatomen sein kann, wobei der Ring noch ein Rest verzweigtes und unverzweigtes C₁-C₆-Alkyl, C₃-C₇-Cycloalk-C₁-C₄-Alkyl, CO-R³¹, COOR³¹ und Phenyl tragen kann, und R¹⁰ Wasserstoff, C₁-C₄-Alkyl oder Phenyl bedeuten, und R³¹ die selbe Bedeutung wie R²¹ annehmen kann,
sowie ihre tautomeren Formen, möglichen enantiomeren und diastereomeren Formen, deren Prodrugs ausgewählt aus der Gruppe der Phosphate, Carbamate von Aminosäuren und Ester der allgemeinen Formel 1, sowie mögliche physiologisch verträgliche Salze.

Bevorzugt werden die Verbindungen der allgemeinen Formel I, worin R¹ Wasserstoff bedeutet.

Bevorzugt werden die Verbindungen der allgemeinen Formel I, worin R² Wasserstoff bedeutet.

Bevorzugt werden die Verbindungen der allgemeinen Formel I, worin R⁴ Wasserstoff bedeutet.

Bevorzugt werden die Verbindungen der allgemeinen Formel I, worin R³ an den Stickstoff von A gebunden ist.

Bevorzugt werden die Verbindungen der allgemeinen Formel I, worin R³ Wasserstoff, C₁-C₆-Alkyl, Benzyl und Phenethyl bedeutet.

Besonders bevorzugt sind die Verbindungen der allgemeinen Formel I, worin R¹, R² und R⁴ Wasserstoff und A Piperidin, das mit der 4-Stellung am Benzimidazol gebunden ist, sind und R³ wasserstoff, C₁-C₆-Alkyl, Benzyl und Phenethyl bedeutet und in 1-Stellung am piperidin-Ring gebunden ist.

Die jeweiligen Bedeutungen von R⁵ bis R¹⁰ sind in den Resten R¹ bis R⁴ unabhängig voneinander.

Die bevorzugte Bedeutung von NR⁸R⁹, NR²⁴R²⁵ und NR³²R³³ als zyklisches Amin sind Piperidin, Pyrrolidin, Piperazin und Homopiperazin. Bevorzugterweise kann bei Piperazin und Homopiperazin der Ring noch ein Rest verzweigtes und unverzweigtes C₁-C₆-Alkyl, C₃-C₇-CyCloalk-C₁-C₄-Alkyl, CO-R⁷ mit R⁷ = R⁴¹ für NR⁸R⁹, R⁷ = R²² für

NR²⁴R²⁵ und R⁷ = R³¹ für NR³²R³³, und Phenyl tragen.

Die bevorzugte Bedeutung von A ist Piperidin, Pyrrolidin. Piperazin, Morpholin oder Homopiperazin.

Besonders bevorzugt werden die Verbindungen der allgemeinen Formel I, worin A Piperazin oder Piperidin bedeutet.

Die Verbindungen der Formel I können als Racemate, als enantiomerenreine Verbindungen oder als Diastereomere eingesetzt werden. Werden enantiomerenreine Verbindungen gewünscht, kann man diese beispielsweise dadurch erhalten, daß man mit einer geeigneten optisch aktiven Base oder Säure eine klassische Racematspaltung mit den Verbindungen der Formel I oder ihren Zwischenprodukten durchführt.

Die gesättigten oder einfach ungesättigten Zyklen A können als cis-Isomere, trans-Isomere oder deren Gemische vorliegen.

Gegenstand der Erfindung sind auch zu Verbindungen der Formel I mesomere oder tautomere Verbindungen.

Ein weiterer Gegenstand der Erfindung sind die physiologisch verträglichen Salze der Verbindungen I, die sich durch Umsatz von Verbindungen I mit einer geeigneten Säure oder Base erhalten lassen. Geeignete Säuren und Basen sind zum Beispiel in Fortschritte der Arzneimittelforschung, 1966, Birkhäuser Verlag, Bd.10, S. 224-285, aufgelistet. Dazu zählen zum Beispiel Salzsäure, Citronensäure, Weinsäure, Milchsäure, Phosphorsäure, Methansulfonsäure, Essigsäure, Ameisensäure, Maleinsäure, Fumarsäure usw. bzw. Natriumhydroxid, Lithiumhydroxid, Kaliumhydroxid und Tris.

Unter Prodrugs werden solche Verbindungen verstanden, die *in vivo* in Verbindungen der allgemeinen Formel I metabolisiert werden. Typische Prodrugs sind Phosphate, Carbamate von Aminosäuren, Ester und andere.

Die Herstellung der erfindungsgemäßen Benzimidazole I kann auf verschiedenen Wegen erfolgen, die im Syntheseschema 1 skizziert wurde.

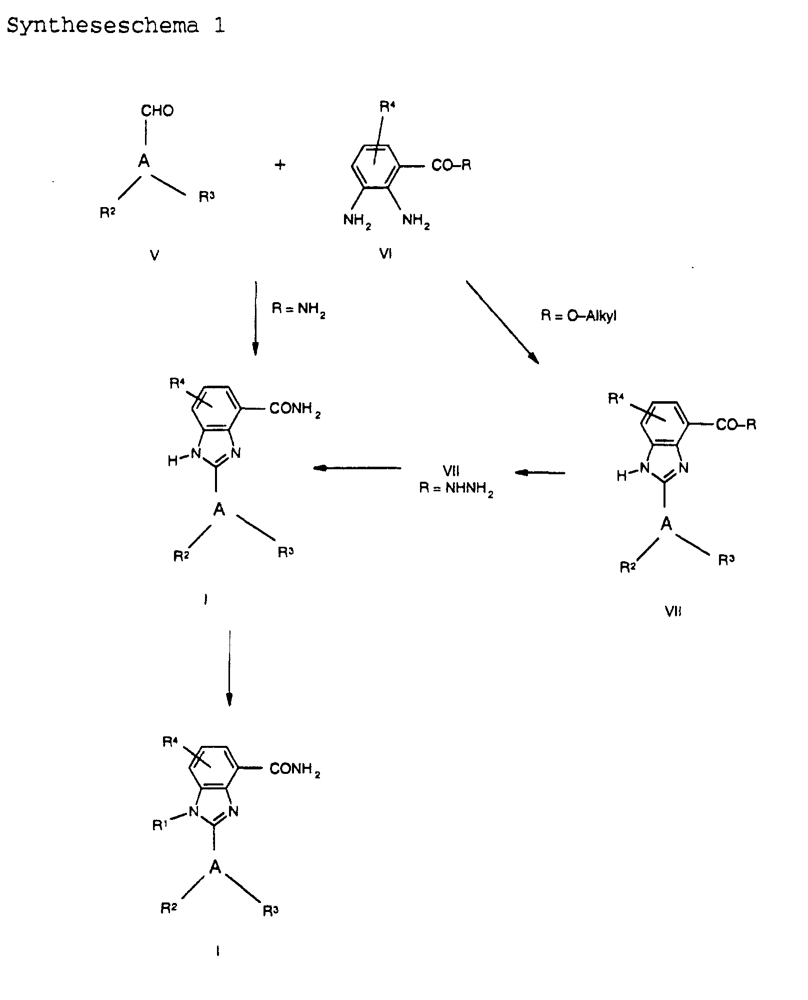

Durch Kondensation des Aldehyds V mit Phenylendiaminen VI erhält man das Benzimidazol I bzw. VII, wobei man bevorzugt in polaren Lösungsmitteln wie Ethanol oder Dimethylformamid und Zusatz von Säuren wie Essigsäure bei erhöhter Temperatur arbeitet, in der Regel 80 bis 120°C. Günstig für die Reaktion ist der Zusatz von schwachen Oxidationsmittel wie Kupfer-II-Salzen, die als wäßrige Lösung zugesetzt werden.

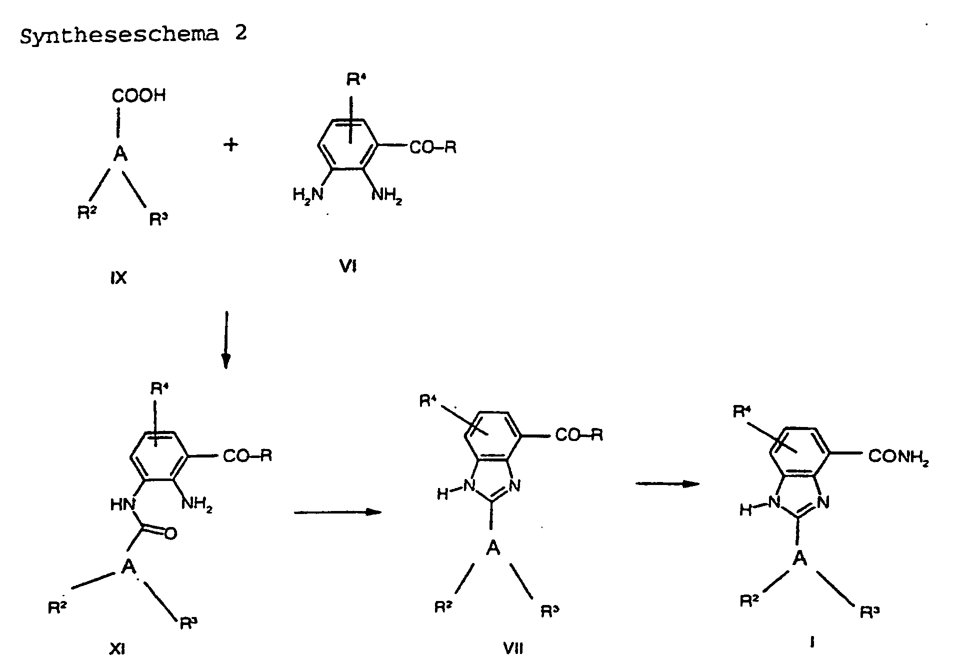

Wenn in dem Phenylendiamin VI R = NH₂ ist, entstehen bei der Kondensation direkt erfindungsgemäße Verbindungen I. Ansonsten kann man, falls R = O-Alkyl ist, diesen Ester mit Ammoniak, bei gegebenenfalls erhöhter Temperatur und erhöhtem Druck, zum Amid I umsetzten. Alternativ kann man den Ester VII mit Hydrazin in polaren Lösungsmitteln wie die Alkohole Butanol und Ethanol oder auch Dimethylformamid, bei erhöhten Temperaturen, vorzugsweise 80 bis 130°C, umsetzten, wobei ein Hydrazid VII (R = NHNH₂) anfällt, das danach noch unter reduktiven Bedingungen, wie mit Raney-Nickel in Alkoholen unter Rückfluß, zum Amid I reduziert werden kann.

Eine Einführung des Restes R1 am Benzimidazol-Rest in I (R1 = H) gelingt unter üblichen Alkylierungsbedingungen. Dabei werden Benzimidazole I mit R¹ - L, wobei L eine Abgangsgruppe darstellt, unter Benutzung einer Base bei 25 bis 150°C, vornehmlich aber bei erhöhter Temperatur wie 60 bis 130°C, alkyliert, wobei das neue Produkt I mit R¹ ≠ Wasserstoff entsteht. Dabei wird in Lösungsmitteln wie zum Beispiel Dimethylformamid, Dimethylsulfoxid, Alkohole, z.B. Ethanol, Ketonen, z.B. Methylethylketon, Aceton, aliphatischen Ethern, z.B. Tetrahydrofuran, und Kohlenwasserstoffen, z.B. Toluol, gearbeitet, wobei man auch Gemische einsetzen kann. Als Base können zum Beispiel Alkoholate, z.B. Natriumethanolat und Kalium-tert.-butanolat, Karbonate, z.B: Kaliumkarbonat, Hydride, z.B. Natriumhydrid, und Hydroxide, z.B. Natriumhydroxid und Kaliumhydroxid, eingesetzt werden. Zudem kann man auch verschiedene Kronenether wie 18-crown-6 in katalytischen Mengen zugeben. Weiterhin kann man unter Phasentransferbedingungen arbeiten (Methoden siehe R.C. Larock, Comprehensive Organic Transformations, 1989, S. 445f.). Als Abgangsgruppe L kann man Halogenide, z.B. Brom, Chlor und Jod, oder auch zum Beispiel Tosylate oder Mesylate einsetzen.

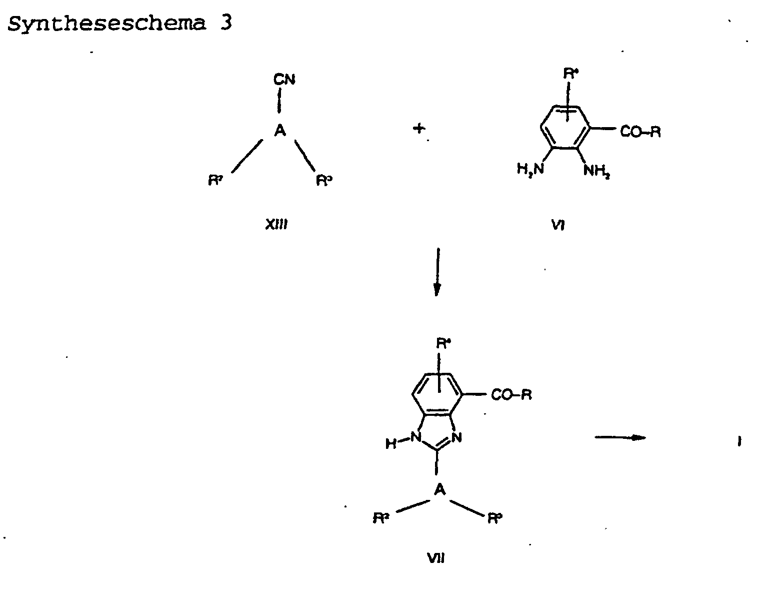

Alternativ zu den im Schema 1 gezeigten Aldehyden V kann man auch Säuren wie IX (siehe Schema 2) oder Nitrite wie XIII (siehe Schema 3) anstelle des Aldehyds einsetzen. Die Herstellung dieser Derivate erfolgt analog zur Herstellung der substituierten Aldehyde V. Ausgehend von IX erfolgt die Kondensation zu VII in zwei Stufen. Zuerst wird die Säure IX mit dem Anilin VI in einer peptidartigen Kupplung zum Amid XI umgesetzt. Dabei arbeitet man nach üblichen Bedingungen, die zum Beispiel im Houben-Weyl, Methoden der Organischen Chemie, 4. Aufl., E5, Kap. V bzw. C.R. Larock, Comprehensive Organic Transformations, VCH Publisher, 1989, Seite 972f. aufgelistet sind. Der Ringschluß zum Benzimidazol erfolgt danach bei erhöhter Temperatur, zum Beispiel 60 bis 180°C, mit oder ohne Lösungsmitteln wie Dimethylformamid, unter Zusatz von Säuren wie Essigsäure oder direkt in Essigsäure selbst.

Die Reaktion des Phenylendiamins VI mit einem Nitril XIII erfolgt ebenfalls unter üblichen Bedingungen. Dabei kann man in Lösungsmitteln wie Dimethylformamid unter Zusatz von Säuren bei erhöhter Temperatur wie 60 bis 200°C arbeiten. Allerdings kann man auch die üblichen Methoden zur Herstellung von Amidinen aus Nitrilen anwenden, wie sie in J. Amer. Chem. Soc. 1957, 427 und J. Org Chem. 1987, 1017, beschrieben sind.

Die in der vorliegenden Erfindung enthaltenen substituierten Benzimidazole I stellen Inhibitoren des Enzyms Poly(ADP-ribose) polymerase oder PARP (EC 2.4.2.30) dar.

Die inhibitorische Wirkung der substituierten Benzimidazole I wurde mit einem in der Literatur bereits bekannten Enzymtest ermittelt, wobei als Wirkmaßstab ein Kᵢ-Wert ermittelt wurde. Die Benzimidazole I wurden in dieser Weise auf Hemmwirkung des Enzyms Poly(ADP-ribose)polymerase oder PARP (EC 2.4.2.30) gemessen.

Die substituierten Benzimidazole der allgemeinen Formeln I stellen Inhibitoren der Poly(ADP-ribose)polymerase (PARP) bzw. wie es auch genannt wird Poly(ADP-ribose)synthase (PARS) dar und können somit zur Behandlung und Prophylaxe von Krankheiten, die mit einer erhöhten Enzymaktivität dieser Enzyme verbunden sind, dienen.

Die Verbindungen der Formeln I können zur Herstellung von Arzneimitteln zur Behandlung von Schädigungen nach Ischämien und zur Prophylaxe bei erwarteten Ischämien verschiedener Organe eingesetzt werden.

Die vorliegenden Benzimidazole der allgemeinen Formel I können danach zur Behandlung und Prophylaxe von neurodegenerativen Krankheiten, die nach Ischämie, Trauma (Schädel-Hirntrauma), Massenblutungen. Subarachnoidal-Blutungen und Stroke auftreten, und von neurodegenerativen Krankheiten wie multipler Infarkt-Dementia, Alzheimer Krankheit, Parkinson Krankheit, Huntington Krankheit und von Epilepsien, insbesondere von generalisierten epileptischen Anfällen, wie zum Beispiel Petit mal und tonisch-clonische Anfälle und partiell epileptischen Anfällen, wie Temporal Lope, und komplex-partiellen Anfällen, und weiterhin zur Behandlung und Prophylaxe von Schädigungen des Herzens nach cardialen Ischämien und Schädigungen der Nieren nach renalen Ischämien, zum Beispiel der akuten Niereninsuffizienz, des akuten Nierenversagens, Schädigungen, die durch medikamentöse Therapie verursacht werden, wie z.B. während der Cyclosporin-Therapie oder von Schädigungen, die während und nach einer Nierentransplantation auftreten, dienen. Weiterhin können die Verbindungen der allgemeinen Formel I zur Behandlung des akuten Myocardinfarkts und Schädigungen, die während und nach dessen medikamentöser Lyse auftreten (zum Beispiel mit TPA, Reteplase, Streptokinase oder mechanisch mit einem Laser oder Rotablator) und von Mikroinfarkten wie z.B. während und nach Herzklappenersatz, Aneurysmenresektionen und Herztransplantationen dienen. Ebenfalls können die vorliegenden Benzimidazole I zur Behandlung einer Revascularisation kritisch verengter Koronaraterien, zum Beispiel bei der PCTA und Bypass-Operationen, und kritisch verengter peripherer Arterien, zum Beispiel Beinarterien, dienen. Zudem können die Benzimidazole I bei der Chemotherapie von Tumoren und deren Metastasierung nützlich sein und zur Behandlung von Entzündungen und rheumatischen Erkrankungen, wie z.B. rheumatischer Arthritis dienen. Außerdem können die Verbindungen der Formel I zur Behandlung von Diabetes mellitus dienen oder zur Behandlung von Sepsis und Multiorganversagen, wie z.B. während des spetischen Schocks und "adulte respiratory distress-syndrom" (ARDS, Schocklunge).

Die erfindungsgemäßen Arzneimittelzubereitungen enthalten neben den üblichen Arzneimittelhilfsstoffen eine therapeutisch wirksame Menge der Verbindungen I.

Für die lokale äußere Anwendung, zum Beispiel in Puder, Salben oder Sprays, können die Wirkstoffe in den üblichen Konzentrationen enthalten sein. In der Regel sind die Wirkstoffe in einer Menge von 0,001 bis 1 Gew.-%, vorzugsweise 0,001 bis 0,1 Gew.-% enthalten.

Bei der inneren Anwendung werden die Präperationen in Einzeldosen verabreicht. In einer Einzeldosis werden pro kg Körpergewicht 0,1 bis 100 mg gegeben. Die Zubereitung können täglich in einer oder mehreren Dosierungen je nach Art und Schwere der Erkrankungen verabreicht werden.

Entsprechend der gewünschten Applikationsart enthalten die erfindungsgemäßen Arzneimittelzubereitungen neben dem Wirkstoff die üblichen Trägerstoffe und Verdünnungsmittel. Für die lokale äußere Anwendung können pharmazeutisch-technische Hilfsstoffe, wie Ethanol, Isopropanol, oxethyliertes Ricinusöl, oxethyliertes Hydriertes Ricinusöl, Polyacrylsäure, Polyethylenglykol, Polyethylenglykostearat, ethoxylierte Fettalkohole, Paraffinöl, Vaseline und Wollfett, verwendet werden. Für die innere Anwendung eignen sich zum Beispiel Milchzucker, Propylenglykol, Ethanol, Stärke, Talk und Polyvinylpyrrolidon.

Ferner können Antioxidationsmittel wie Tocopherol und butyliertes Hydroxyanisol sowie butyliertes Hydroxytoluol, geschmacksverbessernde Zusatzstoffe, Stabilisierungs-, Emulgier- und Gleitmittel enthalten sein.

Die neben dem Wirkstoff in der Zubereitung enthaltenen Stoffe sowie die bei der Herstellung der pharmazeutischen Zubereitungen verwendeten Stoffe sind toxikologisch unbedenklich und mit dem jeweiligen Wirkstoff verträglich. Die Herstellung der Arzneimittelzubereitungen erfolgt in üblicher Weise, zum Beispiel durch Vermischung des Wirkstoffes mit anderen üblichen Trägerstoffen und Verdünnungsmitteln.

Die Arzneimittelzubereitungen können in verschiedenen Applikationsweisen verabreicht werden, zum Beispiel peroral, parenteral wie intravenös durch Infusion, subkutan, intraperitoneal und topisch. So sind Zubereitungsformen wie Tabletten, Emulsionen, Infusions- und Injektionslösungen, Pasten, Salben, Gele, Cremes, Lotionen, Puder und Sprays möglich.

Außer den in den Beispielen genannten Substanzen sind folgende Verbindungen besonders bevorzugt und können gemäß den genannten Herstellungsvorschriften synthetisiert werden:
1. 2-(N(O-tert.-Butyloxycarbonyl)-piperidin-4-yl)-benzimidazol-4-carbonsäureamid
2. 2-(N-Methyl-piperidin-4-yl)-benzimidazol-4-carbonsäureamid
3. 2-(N-iso-Propyl-piperidin-4-yl)-benzimidazol-4-carbonsäureamid
4. 2-(N-Cyclohexyl-piperidin-4-yl)-benzimidazol-4-carbonsäureamid
5. 2-(N-(trans-4-Propyl-cyclohex-1-yl)-piperidin-4-yl)-benzimidazol-4-carbonsäureamid
6. 2-(N-Benzyl-piperidin-4-yl)-benzimidazol-4-carbonsäureamid
7. 2-(N-(2-Phenyl)eth-1-yl)-piperidin-4-yl)-benzimidazol-4-carbo nsäureamid
8. 2-(N-(2(4-Fluorphenyl)eth-1-yl)-piperidin-4-yl)-benzimidazol-4-carbonsäureamid
9. 2-(N-(2(4-Chlorphenyl)eth-1-yl)-piperidin-4-yl)-benzimidazol-4-carbonsäureamid
10. 2-(N-(2(4-Bromphenyl)eth-1-yl)-piperidin-4-yl)-benzimidazol-4-carbonsäureamid
11. 2-(N-(2(4-Iodphenyl)eth-1-yl)-piperidin-4-yl)-benzimidazol-4-carbonsäureamid
12. 2-(N-(2(4-Nitrophenyl)eth-1-yl)-piperidin-4-yl)-benzimidazol-4-carbonsäureamid
13. 2-(N-(2(4-Cyanphenyl)eth-1-yl)-piperidin-4-yl)-benzimidazol-4-carbonsäureamid
14. 2-(N-(2(4-(Trifluormethyl)phenyl)eth-1-yl)-piperidin-4-yl)-benzimidazol-4-carbonsäureamid
15. 2-(N-(2(4-Methylphenyl)eth-1-yl)-piperidin-4-yl)-benzimidazol-4-carbonsäureamid
16. 2-(N-(2(4-Hydroxyphenyl)eth-1-yl)-piperidin-4-yl)-benzimidazol-4-carbonsäureamid
17. 2-(N-(2(4-Methoxyphenyl)eth-1-yl)-piperidin-4-yl)-benzimidazol-4-carbonsäureamid
18. 2-(N-(2(4-(N',N'-Dimethylamino)phenyl)eth-1-yl)-piperidin-4-yl)-benzimidazol-4-carbonsäureamid
19. 2-(N-(2(4-(N'-Acetylamino)phenyl)eth-1-yl)-piperidin-4-yl)-benzimidazol-4-carbonsäureamid
20. 2-(N-(2(4-(N'-Phenylsulfonylamino)phenyl)eth-1-yl)-piperidin-4-yl)-benzimidazol-4-carbonsäureamid
21. 2-(N-(2(4-(Phenylsulfonyl)phenyl)eth-1-yl)-piperidin-4-yl)-benzimidazol-4-carbonsäureamid
22. 2-(N-(2(4-(Methoxycarbonyl)phenyl)eth-1-yl)-piperidin-4-yl)-benzimidazol-4-carbonsäureamid
23. 2-(N-Acetyl-piperidin-3-yl)-benzimidazol-4-carbonsäureamid
24. 2-(N-Propyl-piperidin-3-yl)-benzimidazol-4-carbonsäureamid
25. 2-(N-iso-Propyl-piperidin-3-yl)-benzimidazol-4-carbonsäureamid
26. 2-(N-Cyclohexyl-piperidin-3-yl)-benzimidazol-4-carbonsäureamid
27. 2-(N-(trans-4-Propyl-cyclohex-1-yl)-piperidin-3-yl)-benzimidazol-4-carbonsäureamid
28. 2-(N-(2-Phenyl)eth-1-yl)-piperidin-3-yl)-benzimidazol-4-carbonsäureamid
29. 2-(N-(2(4-Chlorphenyl)eth-1-yl)-piperidin-3-yl)-benzimidazol-4-carbonsäureamid
30. 2-Pyrrolidin-3-yl-benzimidazol-4-carbonsäureamid
31. 2-(N-Acetyl-pyrrolidin-3-yl)-benzimidazol-4-carbonsäureamid
32. 2-(N(O-tert.-Butyloxycarbonyl)-pyrrolidin-3-yl)-benzimidazol-4-carbonsäureamid
33. 2-(N-Propyl-pyrrolidin-3-yl)-benzimidazol-4-carbonsäureamid
34. 2-(N-iso-Propyl-pyrrolidin-3-yl)-benzimidazol-4-carbonsäureamid
35. 2-(N-Cyclohexyl-pyrrolidin-3-yl)-benzimidazol-4-carbonsäureamid
36. 2-(N-(trans-4-Propyl-cyclohex-1-yl)-pyrrolidin-3-yl)-benzimidazol-4-carbonsäureamid
37. 2-(N-Benzyl-pyrrolidin-3-yl)-benzimidazol-4-carbonsäureamid
38. 2-(N-(2-Phenyl)eth-1-yl)-pyrrolidin-3-yl)-benzimidazol-4-carbonsäureamid
39. 2-(N-(2(4-Chlorphenyl)eth-1-yl)-pyrrolidin-3-yl)-benzimidazol-4-carbonsäureamid
40. 2-(N-(2(4-Nitrophenyl)eth-1-yl)-pyrrolidin-3-yl)-benzimidazol-4-carbonsäureamid
41. 2-(N-(2(4-Cyanphenyl)eth-1-yl)-pyrrolidin-3-yl)-benzimidazol-4-carbonsäureamid
42. 2-(N-(2(4-(Trifluormethyl)phenyl)eth-1-yl)-pyrrolidin-3-yl)-benzimidazol-4-carbonsäureamid
43. 2-(N-(2(4-Methylphenyl)eth-1-yl)-pyrrolidin-3-yl)-benzimidazol-4-carbonsäureamid
44. 2-(N-(2(4-Hydroxyphenyl)eth-1-yl)-pyrrolidin-3-yl)-benzimidazol-4-carbonsäureamid
45. 2-(N-(2(4-Methoxyphenyl)eth-1-yl)-pyrrolidin-3-yl)-benzimidazol-4-carbonsäureamid
46. 2-(N-(2(4-(N',N'-Dimethylamino)phenyl)eth-1-yl)-pyrrolidin-3-yl)-benzimidazol-4-carbonsäureamid
47. 2-(N-(2(4-(N'-Acetylamino)phenyl)eth-1-yl)-pyrrolidin-3-yl)-benzimidazol-4-carbonsäureamid
48. 2-(N-(2(4-(N'-Phenylsulfonylamino)phenyl)eth-1-yl)-pyrrolidin-3-yl)-benzimidazol-4-carbonsäureamid
49. 2-(N-(2(4-(Phenylsulfonyl)phenyl)eth-1-yl)-pyrrolidin-3-yl)-benzimidazol-4-carbonsäureamid
50. 2-(N-(2(4-(Methoxycarbonyl)phenyl)eth-1-yl)-pyrrolidin-3-yl)-benzimidazol-4-carbonsäureamid
51. 2-Pyrrolidin-2-yl-benzimidazol-4-carbonsäureamid
52. 2-(N-Acetyl-piperazin-4-yl)-benzimidazol-4-carbonsäureamid
53. 2-(N(O-tert.-Butyloxycarbonyl)-piperazin-4-yl)-benzimidazol-4-carbonsäureamid
54. 2-(N-Methyl-piperazin-4-yl)-benzimidazol-4-carbonsäureamid
55. 2-(N-Propyl-piperazin-4-yl)-benzimidazol-4-carbonsäureamid
56. 2-(N-iso-Propyl-piperazin-4-yl)-benzimidazol-4-carbonsäureamid
57. 2-(N-Cyclohexyl-piperazin-4-yl)-benzimidazol-4-carbonsäureamid
58. 2-(N-(trans-4-Propyl-cyclohex-1-yl)-piperazin-4-yl)-benzimidazol-4-carbonsäureamid
59. 2-(N-Benzyl-piperazin-4-yl)-benzimidazol-4-carbonsäureamid
60. 2-(N-(2-Phenyl)eth-1-yl)-piperazin-4-yl)-benzimidazol-4-carbonsäureamid
61. 2-(N-(2(4-Fluorphenyl)eth-1-yl)-piperazin-4-yl)-benzimidazol-4-carbonsäureamid
62. 2-(N-(2(4-Chlorphenyl)eth-1-yl)-piperazin-4-yl)-benzimidazol-4-carbonsäureamid
63. 2-(N-(2(4-Bromphenyl)eth-1-yl)-piperazin-4-yl)-benzimidazol-4-carbonsäureamid
64. 2-(N-(2(4-Iodphenyl)eth-1-yl)-piperazin-4-yl)-benzimidazol-4-carbonsäureamid
65. 2-(N-(2(4-Nitrophenyl)eth-1-yl)-piperazin-4-yl)-benzimidazol-4-carbonsäureamid
66. 2-(N-(2(4-Cyanphenyl)eth-1-yl)-piperazin-4-yl)-benzimidazol-4-carbonsäureamid
67. 2-(N-(2(4-(Trifluormethyl)phenyl)eth-1-yl)-piperazin-4-yl)-benzimidazol-4-carbonsäureamid
68. 2-(N-(2(4-Methylphenyl)eth-1-yl)-piperazin-4-yl)-benzimidazol-4-carbonsäureamid
69. 2-(N-(2(4-Hydroxyphenyl)eth-1-yl)-piperazin-4-yl)-benzimidazol-4-carbonsäureamid
70. 2-(N-(2(4-Methoxyphenyl)eth-1-yl)-piperazin-4-yl)-benzimidazol-4-carbonsäureamid
71. 2-(N-(2(4-(N',N'-Dimethylamino)phenyl)eth-1-yl)-piperazin-4-yl)-benzimidazol-4-carbonsäureamid
72. 2-(N-(2(4-(N'-Acetylamino)phenyl)eth-1-yl)-piperazin-4-yl)-benzimidazol-4-carbonsäureamid
73. 2-(N-(2(4-(N'-Phenylsulfonylamino)phenyl)eth-1-yl)-piperazin4-yl)-benzimidazol-4-carbonsäureamid
74. 2-(N-(2(4-(Phenylsulfonyl)phenyl)eth-1-yl)-piperazin-4-yl)-benzimidazol-4-carbonsäureamid
75. 2-(N-(2(4-(Methoxycarbonyl)phenyl)eth-1-yl)-piperazin-4-yl)-benzimidazol-4-carbonsäureamid
76. 2-Homopiperazin-4-yl-benzimidazol-4-carbonsäureamid
77. 2-(N-Acetyl-homopiperazin-4-yl)-benzimidazol-4-carbonsäureamid
78. 2-(N(O-tert.-Butyloxycarbonyl)-homopiperazin-4-yl)-benzimidazol-4-carbonsäureamid
79. 2-(N-Methyl-homopiperazin-4-yl)-benzimidazol-4-carbonsäureamid
80. 2-(N-Propyl-homopiperazin-4-yl)-benzimidazol-4-carbonsäureamid
81. 2-(N-iso-Propyl-homopiperazin-4-yl)-benzimidazol-4-carbonsäureamid
82. 2-(N-Cyclohexyl-homopiperazin-4-yl)-benzimidazol-4-carbonsäureamid
83. 2-(N-(trans-4-Propyl-cyclohex-1-yl)-homopiperazin-4-yl)-benzimidazol-4-carbonsäureamid
84. 2-(N-Benzyl-homopiperazin-4-yl)-benzimidazol-4-carbonsäureamid
85. 2-(N-(2-Phenyl)eth-1-yl)-homopiperazin-4-yl)-benzimidazol-4-carbonsäureamid
86. 2-(N-(2(4-Fluorphenyl)eth-1-yl)-homopiperazin-4-yl)-benzimidazol-4-carbonsäureamid
87. 2-(N-(2(4-Chlorphenyl)eth-1-yl)-homopiperazin-4-yl)-benzimidazol-4-carbonsäureamid
88. 2-(N-(2(4-Bromphenyl)eth-1-yl)-homopiperazin-4-yl)-benzimidazol-4-carbonsäureamid
89. 2-(N-(2(4-Iodphenyl)eth-1-yl)-homopiperazin-4-yl)-benzimidazol-4-carbonsäureamid
90. 2-(N-(2(4-Nitrophenyl)eth-1-yl)-homopiperazin-4-yl)-benzimidazol-4-carbonsäureamid
91. 2-(N-(2(4-Cyanphenyl)eth-1-yl)-homopiperazin-4-yl)-benzimidazol-4-carbonsäureamid
92. 2-(N-(2(4-(Trifluormethyl)phenyl)eth-1-yl)-homopiperazin-4-yl)-benzimidazol-4-carbonsäureamid
93. 2-(N-(2(4-Methylphenyl)eth-1-yl)-homopiperazin-4-yl)-benzimidazol-4-carbonsäureamid
94. 2-(N-(2(4-Hydroxyphenyl)eth-1-yl)-homopiperazin-4-yl)-benzimidazol-4-carbonsäureamid
95. 2-(N-(2(4-Methoxyphenyl)eth-1-yl)-homopiperazin-4-yl)-benzimidazol-4-carbonsäureamid
96. 2-(N-(2(4-(N',N'-Dimethylamino)phenyl)eth-1-yl)-homopiperazin-4-yl)-benzimidazol-4-carbonsäureamid
97. 2-(N-(2(4-(N'-Acetylamino)phenyl)eth-1-yl)-homo-piperazin-4-yl)-benzimidazol-4-carbonsäureamid
98. 2-(N-(2(4-(N'-Phenylsulfonylamino)phenyl)eth-1-yl)-homopiperazin-4-yl)-benzimidazol-4-carbonsäureamid
99. 2-(N-(2(4-(Phenylsulfonyl)phenyl)eth-1-yl)-homopiperazin-4-yl)-benzimidazol-4-carbonsäureamid
100.2-(N-(2(4-(Methoxycarbonyl)phenyl)eth-1-yl)-homopiperazin-4-yl)-benzimidazol-4-carbonsäureamid
101.1-Methyl-2-(piperidin-4-yl)-benzimidazol-4-carbonsäureamid
102.2-(N(O-tert.-Butyloxycarbonyl)-piperidin-4-yl)-1-methyl-benzimidazol-4-carbonsäureamid
103.1-Methyl-2-(N-methyl-piperidin-4-yl)-benzimidazol-4-carbonsäureamid
104.1-Methyl-2-(N-iso-Propyl-piperidin-4-yl)-benzimidazol-4-carbonsäureamid
105.2-(N-Benzyl-piperidin-4-yl)-1-methyl-benzimidazol-4-carbonsäureamid
106.1-Methyl-2-(N-(2-phenyl)eth-1-yl)-piperidin-4-yl)-benzimidazol-4-carbonsäureamid
107.2-(N-(2(4-Chlorphenyl)eth-1-yl)-piperidin-4-yl)-1-methylbenzimidazol-4-carbonsäureamid
108.2-(N-Acetyl-piperidin-3-yl)-1-methyl-benzimidazol-4-carbonsäureamid
109.1-Methyl-2-(pyrrolidin-3-yl)-benzimidazol-4-carbonsäureamid
110.2-(N-Acetyl-pyrrolidin-3-yl)-1-methyl-benzimidazol-4-carbonsäureamid
111.2-(N(O-tert.-Butyloxycarbonyl)-pyrrolidin-3-yl)-1-methylbenzimidazol-4-carbonsäureamid
112.1-Methyl-2-(N-methyl-pyrrolidin-3-yl)-benzimidazol-4-carbonsäureamid
113.1-Methyl-2-(N-propyl-pyrrolidin-3-yl)-benzimidazol-4-carbonsäureamid
114.1-Methyl-2-(N-iso-propyl-pyrrolidin-3-yl)-benzimidazol-4-carbonsäureamid
115.2-(N-Benzyl-pyrrolidin-3-yl)-1-methyl-benzimidazol-4-carbonsäureamid
116.1-Methyl-2-(N-(2-phenyl)eth-1-yl)-pyrrolidin-3-yl)-benzimidazol-4-carbonsäureamid
117.2-(N-(2(4-Chlorphenyl)eth-1-yl)-pyrrolidin-3-yl)-1-methylbenzimidazol-4-carbonsäureamid
118.1-Methyl-2-(pyrrolidin-2-yl)-benzimidazol-4-carbonsäureamid
119.2-(N-Acetyl-pyrrolidin-2-yl)-1-methyl-benzimidazol-4-carbonsäureamid
120.1-Methyl-2-piperazin-4-yl-benzimidazol-4-carbonsäureamid
121.2-(N-Acetyl-piperazin-4-yl)-1-methyl-benzimidazol-4-carbonsäureamid
122.2-(N(O-tert.-Butyloxycarbonyl)-piperazin-4-yl)-1-methyl-benzimidazol-4-carbonsäureamid
123.1-Methyl-2-(N-methyl-piperazin-4-yl)-benzimidazol-4-carbonsäureamid
124.1-Methyl-2-(N-propyl-piperazin-4-yl)-benzimidazol-4-carbonsäureamid
125.1-Methyl-2-(N-iso-propyl-piperazin-4-yl)-benzimidazol-4-carbonsäureamid
126.2-(N-Benzyl-piperazin-4-yl)-1-methyl-benzimidazol-4-carbonsäureamid
127.1-Methyl-2-(N-(2-phenyl)eth-1-yl)-piperazin-4-yl)-benzimidazol-4-carbonsäureamid
128.2-(N-(2(4-Chlorphenyl)eth-1-yl)-piperazin-4-yl)-1-methylbenzimidazol-4-carbonsäureamid
129.2-(Homopiperazin-4-yl)-1-methyl-benzimidazol-4-carbonsäureamid
130.2-(N-Acetyl-homopiperazin-4-yl)-1-methyl-benzimidazol-4-carbonsäureamid
131.2-(N(O-tert.-Butyloxycarbonyl)-homopiperazin-4-yl)-1-methylbenzimidazol-4-carbonsäureamid
132.1-Methyl-2-(N-methyl-homopiperazin-4-yl)-benzimidazol-4-carbonsäureamid
133.1-Methyl-2-(N-propyl-homopiperazin-4-yl)-benzimidazol-4-carbonsäureamid
134.1-Methyl-2-(N-iso-propyl-homopiperazin-4-yl)-benzimidazol-4-carbonsäureamid
135.2-(N-Benzyl-homopiperazin-4-yl)-1-methyl-benzimidazol-4-carbonsäureamid
136.1-Methyl-2-(N-(2-phenyl)eth-1-yl)-homopiperazin-4-yl)-benzimidazol-4-carbonsäureamid
137.2-(N-(2(4-Chlorphenyl)eth-1-yl)-homopiperazin-4-yl)-1-methylbenzimidazol-4-carbonsäureamid
138.1-Ethyl-2-(piperidin-4-yl)-benzimidazol-4-carbonsäureamid
139.2-(Piperidin-4-yl)-1-iso-propyl-benzimidazol-4-carbonsäureamid
140.1-(2-(Hydroxy)eth-1-yl)-2-(piperidin-4-yl)-benzimidazol-4-carbonsäureamid
141.1-(2-(Methoxy)eth-1-yl)-2-(piperidin-4-yl)-benzimidazol-4-carbonsäureamid
142.1-(2-(Amino)eth-1-yl)-2-(piperidin-4-yl)-benzimidazol-4-carbonsäureamid
143.1-(2-(N,N-Dimethylamino)eth-1-yl)-2-(piperidin-4-yl)-benzimidazol-4-carbonsäureamid
144.1-(2-(Piperidin-1-yl)eth-1-yl)-2-(piperidin-4-yl)-benzimidazol-4-carbonsäureamid
145.2-(Piperidin-4-yl)-1-(2-(pyrrolidin-1-yl)eth-1-yl)-benzimida-zol-4-carbonsäureamid
146.1-(2-(2-Ethyl-piperidin-1-yl)eth-1-yl)-2-(piperidin-4-yl)-benzimidazol-4-carbonsäureamid
147.1-Ethyl-2-(piperidin-3-yl)-benzimidazol-4-carbonsäureamid
148.2-(Piperidin-3-yl)-1-iso-propyl-benzimidazol-4-carbonsäureamid
149.1-(2-(Hydroxy)eth-1-yl)-2-(piperidin-3-yl)-benzimidazol-4-carbonsäureamid
150.1-(2-(Methoxy)eth-1-yl)-2-(piperidin-3-yl)-benzimidazol-4-carbonsäureamid
151.1-(2-(Amino)eth-1-yl)-2-(piperidin-3-yl)-benzimidazol-4-carbonsäureamid
152.1-(2-(N,N-Dimethylamino)eth-1-yl)-2-(piperidin-3-yl)-benzimidazol-4-carbonsäureamid
153.1-(2-(Piperidin-1-yl)eth-1-yl)-2-(piperidin-3-yl)-benzimidazol-4-carbonsäureamid
154.2-(Piperidin-3-yl)-1-(2-(pyrrolidin-1-yl)eth-1-yl)-benzimidazol-4-carbonsäureamid
155.1-(2-(2-Ethyl-piperidin-1-yl)eth-1-yl)-2-(piperidin-3-yl)-benzimidazol-4-carbonsäureamid
156.1-Ethyl-2-(pyrrolidin-3-yl)-benzimidazol-4-carbonsäureamid
157.1-iso-Propyl-2-(pyrrolidin-3-yl)-benzimidazol-4-carbonsäureamid
158.1-(2-(Hydroxy)eth-1-yl)-2-(pyrrolidin-3-yl)-benzimidazol-4-carbonsäureamid
159.1-(2-(Methoxy)eth-1-yl)-2-(pyrrolidin-3-yl)-benzimidazol-4-carbonsäureamid
160.1-(2-(Amino)eth-1-yl)-2-(pyrrolidin-3-yl)-benzimidazol-4-carbonsäureamid
161.1-(2-(N,N-Dimethylamino)eth-1-yl)-2-(pyrrolidin-3-yl)-benzimidazol-4-carbonsäureamid
162.1-(2-(Piperidin-1-yl)eth-1-yl)-2-(pyrrolidin-3-yl)-benzimidazol-4-carbonsäureamid
163.2-(Pyrrolidin-3-yl)-1-(2-(pyrrolidin-1-yl)eth-1-yl)-benzimidazol-4-carbonsäureamid
164.1-(2-(2-Ethyl-piperidin-1-yl) eth-1-yl)-2-(pyrrolidin-3-yl)-benzimidazol-4-carbonsäureamid
165.1-Ethyl-2-(pyrrolidin-2-yl)-benzimidazol-4-carbonsäureamid
166.1-iso-Propyl-2-(pyrrolidin-2-yl)-benzimidazol-4-carbonsäureamid
167.1-(2-(Hydroxy)eth-1-yl)-2-(pyrrolidin-2-yl)-benzimidazol-4-carbonsäureamid
168.1-(2-(Methoxy)eth-1-yl)-2-(pyrrolidin-2-yl)-benzimidazol-4-carbonsäureamid
169.1-(2-(Amino)eth-1-yl)-2-(pyrrolidin-2-yl)-benzimidazol-4-carbonsäureamid
170.1-(2-(N,N-Dimethylamino)eth-1-yl)-2-(pyrrolidin-2-yl)-benzimidazol-4-carbonsäureamid
171.1-(2-(Piperidin-1-yl)eth-1-yl)-2-(pyrrolidin-2-yl)-benzimidazol-4-carbonsäureamid
172.2-(Pyrrolidin-2-yl)-1-(2-(pyrrolidin-1-yl)eth-1-yl)-benzimidazol-4-carbonsäureamid
173.1-(2-(2-Ethyl-piperidin-1-yl)eth-1-yl)-2-(pyrrolidin-2-yl)-benzimidazol-4-carbonsäureamid
174.1-Ethyl-2-(piperazin-4-yl)-benzimidazol-4-carbonsäureamid
175.1-iso-Propyl-2-(piperazin-4-yl)-benzimidazol-4-carbonsäureamid
176.1-(2-(Hydroxy)eth-1-yl)-2-(piperazin-4-yl)-benzimidazol-4-carbonsäureamid
177.1-(2-(Methoxy)eth-1-yl)-2-(piperazin-4-yl)-benzimidazol-4-carbonsäureamid
178.1-(2-(Amino)eth-1-yl)-2-(piperazin-4-yl)-benzimidazol-4-carbonsäureamid
179.1-(2-(N,N-Dimethylamino)eth-1-yl)-2-(piperazin-4-yl)-benzimidazol-4-carbonsäureamid
180.2-(Piperazin-4-yl)-1-(2-(piperidin-1-yl)eth-1-yl)-benzimidazol-4-carbonsäureamid
181.2-(Piperazin-4-yl)-1-(2-(pyrrolidin-1-yl)eth-1-yl)-benzimidazol-4-carbonsäureamid
182.1-(2-(2-Ethyl-piperidin-1-yl)eth-1-yl)-2-(piperazin-4-yl)-benzimidazol-4-carbonsäureamid
183.1-Ethyl-2-(homopiperazin-4-yl)-benzimidazol-4-carbonSäureamid
184.1-iso-Propyl-2-(homopiperazin-4-yl)-benzimidazol-4-carbonsäureamid
185.1-(2-(Hydroxy)eth-1-yl)-2-(homopiperazin-4-yl)-benzimidazol-4-carbonsäureamid
186.1-(2-(Methoxy)eth-1-yl)-2-(homopiperazin-4-yl)-benzimidazol-4-carbonsäureamid
187.1-(2-(Amino)eth-1-yl)-2-(homopiperazin-4-yl)-benzimidazol-4-carbonsäureamid
188.2-(2-(N,N-Dimethylamino)eth-1-yl)-2-(homopiperazin-4-yl)-benzimidazol-4-carbonsäureamid
189.2-(Homopiperazin-4-yl)-1-(2-(piperidin-1-yl)eth-1-yl)-benzimidazol-4-carbonsäureamid
190.2-(Homopiperazin-4-yl)-1-(2-(pyrrolidin-1-yl)eth-1-yl)-benzimidazol-4-carbonsäureamid
191.1-(2-(2-Ethyl-piperidin-1-yl)eth-1-yl)-2-(homopiperazin-4-yl)-benzimidazol-4-carbonsäureamid
192.1-Ethyl-2-(N-propyl-piperidin-4-yl)-benzimidazol-4-carbonsäureamid
193.1-iso-Propyl-2-(N-propyl-piperidin-4-yl)-benzimidazol-4-carbonsäureamid
194.1-(2-(Hydroxy)eth-1-yl)-2-(N-propyl-piperidin-4-yl)-benzimidazol-4-carbonsäureamid
195.1-(2-(Methoxy)eth-1-yl)-2-(N-propyl-piperidin-4-yl)-benzimidazol-4-carbonsäureamid
196.1-(2-(Amino)eth-1-yl)-2-(N-propyl-piperidin-4-yl)-benzimidazol-4-carbonsäureamid
197.1-(2-(N,N-Dimethylamino)eth-1-yl)-2-(N-propyl-piperidin-4-yl)-benzimidazol-4-carbonsäureamid
198.1-(2-(Piperidin-1-yl)eth-1-yl)-2-(N-propyl-piperidin-4-yl)-benzimidazol-4-carbonsäureamid
199.2-(N-Propyl-piperidin-4-yl)-1-(2-(pyrrolidin-1-yl)eth-1-yl)-benzimidazol-4-carbonsäureamid
200.1-(2-(2-Ethyl-piperidin-1-yl)eth-1-yl)-2-(N-propyl-piperidin-4-yl)-benzimidazol-4-carbonsäureamid
201.1-Ethyl-2-(N-propyl-piperidin-3-yl)-benzimidazol-4-carbonsäureamid
202.1-iso-Propyl-2-(N-propyl-piperidin-3-yl)-benzimidazol-4-carbonsäureamid
203.1-(2-(Hydroxy)eth-1-yl)-2-(N-propyl-piperidin-3-yl)-benzimidazol-4-carbonsäureamid
204.1-(2-(Methoxy)eth-1-yl)-2-(N-propyl-piperidin-3-yl)-benzimi-dazol-4-carbonsäureamid
205.1-(2-(Amino)eth-1-yl)-2-(N-propyl-piperidin-3-yl)-benzimidazol-4-carbonsäureamid
206.1-(2-(N,N-Dimethylamino)eth-1-yl)-2-(N-propyl-piperidin-3-yl)-benzimidazol-4-carbonsäureamid
207.1-(2-(Piperidin-1-yl)eth-1-yl)-2-(N-propyl-piperidin-3-yl)-benzimidazol-4-carbonsäureamid
208.2-(N-Propyl-piperidin-3-yl)-1-(2-(pyrrolidin-1-yl)eth-1-yl)-benzimidazol-4-carbonsäureamid
209.1-(2-(2-Ethyl-piperidin-1-yl)eth-1-yl)-2-(N-propyl-piperidin-3-yl)-benzimidazol-4-carbonsäureamid
210.1-Ethyl-2-(N-propyl-pyrrolidin-3-yl)-benzimidazol-4-carbonsäureamid
211.1-iso-Propyl-2-(N-propyl-pyrrolidin-3-yl)-benzimidazol-4-carbonsäureamid
212.1-(2-(Hydroxy)eth-1-yl)-2-(N-propyl-pyrrolidin-3-yl)-benzimidazol-4-carbonsäureamid
213.1-(2-(Methoxy)eth-1-yl)-2-(N-propyl-pyrrolidin-3-yl)-benzimidazol-4-carbonsäureamid
214.1-(2-(Amino)eth-1-yl)-2-(N-propyl-pyrrolidin-3-yl)-benzimidazol-4-carbonsäureamid
215.1-(2-(N,N-Dimethylamino)eth-1-yl)-2-(N-propyl-pyrrolidin-3-yl)-benzimidazol-4-carbonsäureamid
216.1-(2-(Piperidin-1-yl)eth-1-yl)-2-(N-propyl-pyrrolidin-3-yl)-benzimidazol-4-carbonsäureamid
217.2-(N-Propyl-pyrrolidin-3-yl)-1-(2-(pyrrolidin-1-yl)eth-1-yl)-benzimidazol-4-carbonsäureamid
218.1-(2-(2-Ethyl-piperidin-1-yl)eth-1-yl)-2-(N-propyl-pyrrolidin-3-yl)-benzimidazol-4-carbonsäureamid
219.1-Ethyl-2-(N-propyl-pyrrolidin-2-yl)-benzimidazol-4-carbonsäureamid
220.1-iso-Propyl-2-(N-propyl-pyrrolidin-2-yl)-benzimidazol-4-carbonsäureamid
221.1-(2-(Hydroxy)eth-1-yl)-2-(N-propyl-pyrrolidin-2-yl)-benzimidazol-4-carbonsäureamid
222.1-(2-(Methoxy)eth-1-yl)-2-(N-propyl-pyrrolidin-2-yl)-benzimidazol-4-carbonsäureamid
223.1-(2-(Amino)eth-1-yl)-2-(N-propyl-pyrrolidin-2-yl)-benzimidazol-4-carbonsäureamid
224.1-(2-(N,N-Dimethylamino)eth-1-yl)-2-(N-propyl-pyrrolidin-2-yl)-benzimidazol-4-carbonsäureamid
225.1-(2-(Piperidin-1-yl)eth-1-yl)-2-(N-propyl-pyrrolidin-2-yl)-benzimidazol-4-carbonsäureamid
226.2-(Pyrrolidin-2-yl)-1-(2-(N-propyl-pyrrolidin-1-yl)eth-1-yl)-benzimidazol-4-carbonsäureamid
227.1-(2-(2-Ethyl-piperidin-1-yl)eth-1-yl)-2-(N-propyl-pyrrolidin-2-yl)-benzimidazol-4-carbonsäureamid
228.1-Ethyl-2-(N-propyl-piperazin-4-yl)-benzimidazol-4-carbonsäureamid
229.1-iso-Propyl-2-(N-propyl-piperazin-4-yl)-benzimidazol-4-carbonsäureamid
230.1-(2-(Hydroxy)eth-1-yl)-2-(N-propyl-piperazin-4-yl)-benzimidazol-4-carbonsäureamid
231.1-(2-(Methoxy)eth-1-yl)-2-(N-propyl-piperazin-4-yl)-benzimidazol-4-carbonsäureamid
232.1-(2-(Amino)eth-1-yl)-2-(N-propyl-piperazin-4-yl)-benzimidazol-4-carbonsäureamid
233.1-(2-(N,N-Dimethylamino)eth-1-yl)-2-(N-propyl-piperazin-4-yl)-benzimidazol-4-carbonsäureamid
234.1-(2-(Piperidin-1-yl)eth-1-yl)-2-(N-propyl-piperazin-4-yl)-benzimidazol-4-carbonsäureamid
235.2-(N-Propyl-piperazin-4-yl)-1-(2-(pyrrolidin-1-yl)eth-1-yl)-benzimidazol-4-carbonsäureamid
236.1-(2-(2-Ethyl-piperidin-1-yl)eth-1-yl)-2-(N-propyl-piperazin-4-yl)-benzimidazol-4-carbonsäureamid
237.1-Ethyl-2-(N-propyl-homopiperazin-4-yl)-benzimidazol-4-carbonsäureamid
238.1-iso-Propyl-2-(N-propyl-homopiperazin-4-yl)-benzimidazol-4-carbonsäureamid
239.1-(2-(Hydroxy)eth-1-yl)-2-(N-propyl-homopiperazin-4-yl)-benzimidazol-4-carbonsäureamid
240.1-(2-(Methoxy)eth-1-yl)-2-(N-propyl-homopiperazin-4-yl)-benzimidazol-4-carbonsäureamid
241.1-(2-(Amino)eth-1-yl)-2-(N-propyl-homopiperazin-4-yl)-benzimidazol-4-carbonsäureamid
242.1-(2-(N,N-Dimethylamino)eth-1-yl)-2-(N-propyl-homopiperazin-4-yl)-benzimidazol-4-carbonsäureamid
243.1-(2-(Piperidin-1-yl)eth-1-yl)-2-(N-propyl-homopiperazin-4-yl)-benzimidazol-4-carbonsäureamid
244.2-(N-propyl-Homopiperazin-4-yl)-1-(2-(pyrrolidin-1-yl)eth-1-yl)-benzimidazol-4-carbonsäureamid
245.1-(2-(2-Ethyl-piperidin-1-yl)eth-1-yl)-2-(N-propyl-homopiperazin-4-yl)-benzimidazol-4-carbonsäureamid
246.6-Chlor-2-(piperidin-4-yl)-benzimidazol-4-carbonsäureamid
247.6-Chlor-2-(piperidin-3-yl)-benzimidazol-4-carbonsäureamid
248.6-Chlor-2-(pyrrolidin-3-yl)-benzimidazol-4-carbonsäureamid
249.6-Chlor-2-(piperazin-4-yl)-benzimidazol-4-carbonsäureamid
250.6-Chlor-2-(homopiperazin-4-yl)-benzimidazol-4-carbonsäureamid
251.6-Ethyl-2-(piperidin-4-yl)-benzimidazol-4-carbonsäureamid
252.6-Ethyl-2-(piperidin-3-yl)-benzimidazol-4-carbonsäureamid
253.6-Ethyl-2-(pyrrolidin-3-yl)-benzimidazol-4-carbonsäureamid
254.6-Ethyl-2-(piperazin-4-yl)-benzimidazol-4-carbonsäureamid
255.6-Ethyl-2-(homopiperazin-4-yl)-benzimidazol-4-carbonsäureamid
256.6-Amino-2-(piperidin-4-yl)-benzimidazol-4-carbonsäureamid
257.6-Amino-2-(piperidin-3-yl)-benzimidazol-4-carbonsäureamid
258.6-Amino-2-(pyrrolidin-3-yl)-benzimidazol-4-carbonsäureamid
259.6-Amino-2-(piperazin-4-yl)-benzimidazol-4-carbonsäureamid
260.6-Amino-2-(homopiperazin-4-yl)-benzimidazol-4-carbonsäureamid
261.2-(Piperidin-4-yl)-6-(pyrrolidin-1-yl)-benzimidazol-4-carbonsäureamid
262.2-(Piperidin-3-yl)-6-(pyrrolidin-1-yl)-benzimidazol-4-carbonsäureamid
263.2-(Pyrrolidin-3-yl)-6-(pyrrolidin-1-yl)-benzimidazol-4-carbonsäureamid
264.2-(Piperazin-4-yl)-6-(pyrrolidin-1-yl)-benzimidazol-4-carbonsäureamid
265.2-(Homopiperazin-4-yl)-6-(pyrrolidin-1-yl)-benzimidazol-4-carbonsäureamid
266.2-(3-Methyl-piperidin-4-yl)-benzimidazol-4-carbonsäureamid
267.2-(3-Cyclohexyl-piperidin-4-yl)-benzimidazol-4-carbonsäureamid
268.2-(2-Cyclohexyl-piperidin-4-yl)-benzimidazol-4-carbonsäureamid
269.2-(3-Phenyl-piperidin-4-yl)-benzimidazol-4-carbonsäureamid
270.2-(4-Phenyl-piperidin-4-yl)-benzimidazol-4-carbonsäureamid
271.2-(2-(Hydroxycarbonyl)-piperidin-4-yl)-benzimidazol-4-carbonsäureamid
272.2-(2-(Ethoxycarbonyl)-piperidin-4-yl)-benzimidazol-4-carbonsäureamid
273.2-(2-(Cyclohexyloxycarbonyl)-piperidin-4-yl)-benzimidazol-4-carbonsäureamid
274.2-(2-(Benzyloxycarbonyl)-piperidin-4-yl)-benzimidazol-4-carbonsäureamid
275.2-(2-(Phenyloxycarbonyl)-piperidin-4-yl)-benzimidazol-4-carbonsäureamid

### Beispiel 1

### 2-Piperidin-4-yl-benzimidazol-4-carbonsäureamid x 2 HCl

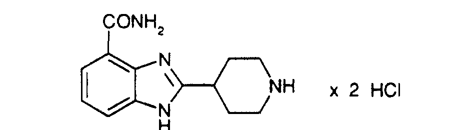

a) N(2-Amino-3-ethoxycarbonyl)-1-(tert.-butyloxycarbonyl)-piperidin-4-carbonsäureanilid
   5,5 g (24 mMol) 1-(tert.-Butyloxycarbonyl)piperidin-4-carbonsäure und 4,3 g (24 mMol) 2,3-Diaminobenzoesäureethylester wurden mit 6,0 g (60 mMol) Triethylamin und 3,2 g (24 mMol) 1-Hydroxybenzotriazol in 100 ml wasserfreiem Tetrahydrofuran gelöst. Bei 0°C wurden anschließend 4,6 g (24 mMol) N'-(3-Dimethylaminopropyl)-N-ethylcarbodiimid zugegeben und alles für 1h gerührt. Danach wurde noch für 24 h bei Raumtemperatur gerührt. Das Reaktionsgemisch wurde im Vakuum eingeengt und der erhaltene Rückstand zwischen Essigester und wäßriger Natriumhydrogenkarbonat-Lösung verteilt. Die Essigester-Phase wurde noch mit 5 %iger wäßriger ZitronensäureLösung gewaschen, getrocknet und im Vakuum eingeengt. Man erhielt 8,4 g des Produktes.
b) 2(1-(tert.-Butyloxycarbonyl)piperidin-4-yl)-benzimidazol-4-carbonsäureethylester
   8,1 g der Zwischenverbindung 1a wurden in 100 ml konzentrierter Essigsäure für 30 Minuten unter Rückfluß gekocht. Anschließend wurde alles im Vakuum eingeengt und der Rückstand zwischen Essigester und Wasser verteilt. Die Essigester-Phase wurde noch mit wäßriger Natriumhydrogenkarbonat-Lösung und Wasser gewaschen und anschließend im Vakuum eingeengt. Man erhielt 4,6 g des Produktes.
c) 2-Piperidin-4-yl-benzimidazol-4-carbonsäureethylester x 2 HCl
   3,7 g (9,9 mMol) der Zwischenverbindung 1b wurden in 50 ml 4M Lösung von Chlorwasserstoff in Dioxan gegeben und für 1 h bei Raumtemperatur gerührt. Anschließend wurde der Ansatz mit viel Ether verdünnt und der entstandene Niederschlag abgesaugt. Man erhielt 3,2 g des Produktes.
d) 2-Piperidin-4-yl-benzimidazol-4-carbonsäurehydrazid
   2,7 g (7,8 mMol) der Zwischenverbindung 1c und 2,7 g (54 mMol) Hydrazin wurden in 30 ml n-Butanol für 15 h unter Rückfluß gekocht. Anschließend wurde alles im Vakuum eingeengt und der erhaltene Rückstand zwischen Essigester und wäßriger Natriumhydrogenkarbonat-Lösung verteilt. Die organische Phase wurde abgetrennt, getrocknet und im Vakuum eingeengt. Man erhielt 0,9 g des Produktes.
e) 2-Piperidin-4-yl-benzimidazol-4-carbonsäureamid x 2 HCl
   Zu 0,8 g (3,1 mMol) der Zwischenverbindung 1d in 20 ml Dimethylformamid wurden ca. 2,4 g Raney-Nickel in 20 ml Wasser gegeben und alles für 8 Stunden auf 100°C erwärmt. Anschließend wurde das Reaktionsgemisch filtriert. Der Rückstand wurde in Ethanol aufgenommen und ein Rohprodukt durch Zugabe von Ether gefällt. Der Niederschlag wurde in Isopropanol gelöst und mit einer Lösung von Chlorwasserstoff in Isopropanol versetzt. Der entstandene Niederschlag wurde abgesaugt. Man erhielt 0,52 g des Produktes.
   ¹H-NMR (D₆-DMSO). δ = 1,8-2,3 (4H), 2,8-3,5 (5H), 7,2 (1H), 7,7 (1H), 7,8 (1H), 8,5 (breit) und 9,2 (breit) ppm.

### Beispiel 2

### 2-Piperidin-4-yl-benzimidazol-4-carbonsäureamid

Das Beispiel wurde analog dem Beispiel 1 hergestellt.
¹H-NMR (D₆-DMSO). δ = 1.7(1H), 1.9-2.2(4H), 2.75(1H), 3.8(1H), 7.2(1H), 7.6(1H), 7.8(1H) und 9.3(breit)ppm.

### Beispiel 3

### 2-(N-Acetyl-piperidin-4-yl)-benzimidazol-4-carbonsäureamid

a) 2-(N-Acetyl-piperidin-4-yl)-benzimidazol-4-carbonsäuremethylester
   3.3 g (19,9 mMol) 2,3-Diaminobenzoesäuremethylester wurden in 100 ml Methanol gelöst und bei Raumtemperatur wurde eine Lösung aus 4,0 g (25,8 mMol) N-Acetylpiperidin-4-aldehyd in 100 ml Methanol zugetropft. Alles wurde für ca. 10 Minuten bei Raumtemperatur gerührt. Danach wurden 5,2 g (25,8 mMol) Kupfer-II-Azetat, das in 100 ml Wasser gelöst wurde, zugetropft und alles für 30 Minuten unter Rückfluß gekocht. Nach dem Abkühlen gab man vorsichtig 25 ml konzentrierte Salzsäure zu und erwärmte erneut alles auf Rückfluß. Jetzt wurden 7,15 g (29,8 mMol) Natriumsulfid Nonahydrat, gelöst in 100 ml Wasser, hinzugetropft und alles für weitere 10 Minuten gekocht. Nach dem Abkühlen wurde die Reaktionslösung im Vakuum eingeengt. Der erhaltene Rückstand wurde in Wasser dispergiert und filtriert. Das Filtrat wurde mit wäßriger Natriumhydrogenkarbonat-Lösung alkalisch gestellt und mehrmals mit Essigester extrahiert. Die vereinigten organischen Phasen wurden noch mit Wasser gewaschen, getrocknet und im Vakuum eingeengt. Man erhielt 4,5 g des Produktes.
b) 2-(N-Acetyl-piperidin-4-yl)-benzimidazol-4-carbonsäurehydrazid
   4,3 g (14,9 mMol) des Zwischenproduktes 3a wurden mit 3,7 g (74,3 mMol) Hydrazin Hydrat in 100 ml Ethanol für 2,5 Stunden unter Rückfluß gekocht. Anschließend wurde alles im Vakuum eingeengt, wobei man ein Rohrprodukt erhielt, das direkt im folgenden Reaktionsschritt eingesetzt wurde.
c) 2-(N-Acetyl-piperidin-4-yl)-benzimidazol-4-carbonsäureamid
   Zu einem Gemisch aus 100 ml Dimethylformamid und 50 ml Wasser wurden 5 g Raney-Nickel gegeben. Bei Raumtemperatur wurden anschließend der Rückstand aus dem Reaktionsschritt 3b, gelöst mit Wasser, vorsichtig zugetropft, so daß die beobachtete Gasentwicklung kontrolliert werden kann. Danach wurde alles für 2 Stunden auf 100°C erwärmt. Nach dem Abkühlen wurde filtriert und das Filtrat im Vakuum eingeengt. Der erhaltene Rückstand wurde in wenig Methylenchlorid aufgenommen und durch vorsihctige Zugabe von Ether wurde das Produkt ausgefällt. Man erhielt 3,2 g des Produktes.
   ¹H-NMR (D₆-DMSO). δ = 1.8-2.3(4H), 2.8-3.5(5H), 7.2(1H), 7.7(1H), 7.8(1H), 8.5(breit) und 9.2(Breit)ppm.

### Beispiel 4

### 2-(N-Propyl-piperidin-4-yl)-benzimidazol-4-carbonsäureamid

0,25 g (1 mMol) des Produktes aus Beispiel 2, 59 mg (1 mMol) n-Propanal und 125 µL (2 mMol) Essigsäure wurden in 25 ml Ethanol gelöst. Danach wurden bei Raumtemperatur 64 mg (1 mMol) Natriumcyanoborhydrid zugefügt und alles für 16 Stunden gerührt. Die Reaktionslösung wurde im Vakuum eingeengt und der Rückstand zwischen Methylenchlorid und wäßriger Natriumhydrogenkarbonat-Lösung verteilt. Die organische Phase wurde mit Wasser gewaschen, abgetrennt, getrocknet und i Vakuum eingeengt. Der erhaltene Rückstand wurde chromatographisch mit dem Fließmittel Essigester/ Methanol = 4/1 gereinigt, wobei man 0,07 g des Produktes erhielt.
¹H-NMR (D₆-DMSO). δ = 0.9(3H), 1.5(2H), 1.9(2H), 2.3(2H), 2.9(2H), 3.3(1H), 7.25(1H), 7.6(1H), 7.8(1H), 9,3(1H) und 12.8(1H)ppm.

### Beispiel 5

### 2-Piperidin-3-yl-benzimidazol-4-carbonsäureamid x 2 HCl

1,3 g (3,8 mMol) des Produktes aus Beispiel 6 wurden in 20 ml Isopropanol gelöst und mit 50 ml isopropanolische Hydrochlorid-Lösung versetzt. Alles wurde für 1 Stunde bei Raumtemperatur gerührt. Der entstandene Niederschlag wurde abgesaugt, wobei man 1,1 g des Produktes erhielt.
¹H-NMR (D₆-DMSO). δ = 1.95-2.3(3H), 2.45(1H), 3.2(1H), 3.5(1H), 3.9(1H), 7.6(1H) und 7.95(2H)ppm.

### Beispiel 6

### 2-(N(O-tert.-Butyloxycarbonyl)-piperidin-3-yl)-benzimidazol-4-carbonsäureamid

a) 2-Amino-3(N(O-tert.-butyloxycarbonyl)-piperidin-3-yl)amidobenzoesäureethylester
   4 g (17,4 mMol) N(O-tert.-Butlyoxycarbonyl)-piperidin-3-carbonsäure und 4,8 ml (34,9 mMol) Triethylamin wurden in 100 ml wasserfreiem Tetrahydrofuran gelöst. Bei -10°C wurden anschließend 1,7 ml (17,4 mMol) Chlorameisensäureethylester, gelöst in 10 ml wasserfreiem Tetrahydrofuran, zugetropft. Alles wurde noch für 1 h bei 0°C gerührt. Danach wurden wieder bei -10°C 2,9 g (17,4 mMol) 2,3-Diaminobenzoesäuremethylester zugefügt und alles für 12 h bei Raumtemperatur gerührt. Die Reaktionslösung wurde im Vakuum eingeengt und der erhaltene Rückstand zwischen Essigester und Wasser verteilt. Die organische Phase wurde noch mit wäßriger Natriumhydrogenkarbonat-Lösung und Wasser gewaschen, getrocknet und im Vakuum eingeengt. Man erhielt 5,5 g des Produktes.
b) 2-(N(O-tert.-Butyloxycarbonyl)-piperidin-3-yl)-benzimidazol-4-carbonsäureethylester
   5,4 g (14,3 mMol) des Produktes aus 6a wurden in 100 ml Essigsäure für 75 Minuten unter Rückfluß gekocht. Nach dem Abküjhlen wurde alles im Vakuum eingeengt und der anfallende Rückstand chromatographisch mit dem Fließmittel Essigester/ Heptan = 1/1 gereinigt. Man erhielt 2,7 g des Produktes.
c) 2-(N(O-tert.-Butyloxycarbonyl)-piperidin-3-yl)-benzimidazol-4-carbonsäurehydrazid
   2,3 g (6,4 mMol) des Produktes aus 6b wurden mit 1,6 g (32 mMol) Hydrazin Hydrat in 20 ml Ethanol für 2,5 Stunden unter Rückfluß gekocht. Nach dem Abkühlen wurde alles im Vakuum eingeengt. Der Rückstand wurde mit Wasser behandelt, wobei ein Niederschlag anfällt, der abgesaugt und getrocknet wurde. Man erhielt 1,6 g des Produktes.
d) 2-(N(O-tert.-Butyloxycarbonyl)-piperidin-3-yl)-benzimidazol-4-carbonsäureamid
   1,6 g des Produktes aus 6c wurden analog der Vorschrift aus 3c umgesetzt. Man erhielt 1,3 g des Produktes.
   ¹H-NMR (D₆-DMSO). δ = 1.4(1H), 1.5(1H), 2.9(1H), 3.1(1H), 3.9(1H), 4.2(1H), 7.3(1H), 7.7(1H), 7.8(1H), 9.1(breit) und ca. 13(breit)ppm.

Analog den Beispielen 1 bis 6 wurden die in den folgenden Beispielen genannten Substanzen hergestellt:

### Beispiel 7

### 2(N-Benzylpiperidin-3-yl)-benzimidazol-4-carbonsäureamid

¹H-NMR (D₆-DMSO) : δ = 1,6-1,8(3H), 2,1(2H), 2,3(2H), 2,8(1H), 3,1(1H), 3,2(1H), 3,5(2H), 7,2-7,4(6H), 7,6(2H), 7,8(2H) und 9,2(breit) ppm.

### Beispiel 8

### 2(N-Methylpiperidin-3-yl)-benzimidazol-4-carbonsäureamid x 2 HCl

¹H-NMR (D₂O) : δ = 2,1(2H), 2,3(1H), 2,5(1H), 3,1(3H), 3,2(1H), 3,5(1H), 3,7(1H), 4,0(2H), 7,7(1H) und 8,0(2H)ppm.

### Beispiel 9

### 2-Piperazin-4-yl-benzimidazol-4-carbonsäureamid

¹H-NMR (D₆-DMSO) : δ = 2,5(4H), 3,3(4H), 7,2(1H), 7,6-7,7(2H), 7,8(1H) und 9,3(1H) ppm.

### Beispiel 10

### 2(N-Propylpiperidin-3-yl)-benzimidazol-4-carbonsäureamid x 2 HCl

¹H-NMR (D₆-DMSO) : δ = 0,9(3H), 1,5(2H), 1,9(2H), 2,0(4H), 2,3(2H), 2,9(3H), 7,2(1H), 7,6(2H), 7,8(1H) und 9,3(breit) ppm.

### Beispiel 11

### 2(N(3-Phenylprop-1-yl)-piperidin-3-yl)-benzimidazol-4-carbonsäureamid x 2HCl

¹H-NMR (D₆-DMSO) : δ = 2,0-2,5(6H), 2,8(2H), 3,1(1H), 3,2-3,4(3H), 3,7(1H), 3,8-4,0(2H), 7,3-7,5(5H), 7,7(1H) und 8,0(2H) ppm.

### Beispiel 12

### 2(N-Benzoylpiperidin-3-yl)-benzimidazol-4-carbonsäureamid

¹H-NMR (CF₃COOD) : δ = 1,9(1H), 2,6(1H), 3,8(1H), 3,9-4,2(4H), 4,3(1H), 4,8(1H) und 7,5-8,2(8H) ppm.

### Beispiel 13

### 2(N-Benzylpiperidin-4-yl)-benzimidazol-4-carbonsäureamid x 2 HCl

¹H-NMR (D₂O) : δ = 2,3(2H), 2,6(2H), 3,3(2H), 3,8(3H), 4,5(2H) und 7,5-8,0(8H) ppm.

### Beispiel 14

### 2(1(1-Methylpiperidin-4-yl)piperidin-4-yl)-benzimidazol-4-carbonsäureamid x 3 HCl

¹H-NMR (D₆-DMSO) : δ = 1,4(2H), 1,6-2,0(6H), 2,0-2,4(7H), 2,7-3,0(6H), 7,2(1H), 7,7(2H), 7,8(1H) und 9,4(breit) ppm.

### Beispiel 15

### 2(N-n-Pentylpiperidin-4-yl)-benzimidazol-4-carbonsäureamid

¹H-NMR (D₆-DMSO) : δ = 0,9(3H), 1,2-1,5(6H), 1,7-2,1(6H), 2,3(2H), 2,8-3,0(4H), 7,3(1H), 7,6-7,8(3H), 9,4(1H) und 12,8(breit) ppm.

### Beispiel 16

### 2(N-Isobut-1-yl-piperidin-4-yl)-benzimidazol-4-carbonsäureamid

¹H-NMR (D₆-DMSO) : δ = 0,9(6H), 1,8-2,1(10H), 2,9(2H), 7,2(1H), 7,6(2H), 7,8(1H), 9,2(1H) und 12,5(breit) ppm.

### Beispiel 17

### 2(N-n-Butylpiperidin-4-yl)-benzimidazol-4-carbonsäureamid x HCl

¹H-NMR (D₆-DMSO) : δ = 0,9(3H), 1,3(2H), 1,7(2H), 2,2-2,4(4H), 3,0-3,2(4H), 3,4-3,6(3H), 7,5(1H), 7,8-8,0(2H), 8,0(1H), 8,7(breit) und 10,9(breit) ppm.

### Beispiel 18

### 2(N-(3-Methyl-but-1-yl)piperidin-4-yl)-benzimidazol-4-carbonsäureamid x HCl

¹H-NMR (D₆-DMSO) : δ = 0,9(6H), 1,7(3H), 2,2-2,4(4H), 3,1(4H), 3,3(1H), 3,7(2H), 7,5(1H), 7,8-8,0(3H), 8,7(breit) und 10,5(breit) ppm.

### Beispiel 19

### 2(1,4-Dimethylpiperazin-2-yl)-benzimidazol-4-carbonsäureamid x 2 HCl

¹H-NMR (D₆-DMSO) : δ = 2,5 (3H), 2,9 (3H), 3,3-3,8 (5H), 3,9 (1H), 5,0 (1H), 7,4 (1H), 7,7 (1H), 7,8 (1H), 7,9 (1H) und 8,6 (breit) ppm.

### Beispiel 20

### 2-Piperazin-2-yl-benzimidazol-4-carbonsäureamid x 2 HCl

1,83 g (3,67 mmol) des Produktes aus Beispiel 23 wurden in 250 ml Methanol mit 1 g 10 % Palladium auf Kohle vorgelegt und mit ca. 165 ml Wasserstoff hydriert. Der Katalysator wurde abgesaugt und das Filtrat wurde eingeengt. Der Rückstand wurde in 20 ml Isopropanol gelöst und mit 50 ml isopropanolischer Hydrochlorid-Lösung versetzt. Der entstandene Niederschlag wurde abgesaugt, wobei man 1,1 g des Produktes erhielt.
¹H-NMR (D₆-DMSO) : δ = 3,2-3,7(5H), 4,0(1H), 5,2(1H), 7,4(1H), 7,8(1H), 7,9(1H) und 10,2(breit) ppm.

### Beispiel 21

### 2(N-Isopropylpiperidin-4-yl)-benzimidazol-4-carbonsäureamid x HCl

¹H-NMR (D₆-DMSO) : δ = 1,25(6H), 2,3(4H), 3,1(1H), 3,4-3,6(4H), 3,7(1H), 7,5(1H), 7,7-8,0(3H), 8,7(1H) und 10,7(breit) ppm.

### Beispiel 22

### 2(4-(2-Ethyl-prop-1-yl)piperidin-4-yl)-benzimidazol-4-carbonsäureamid

### Beispiel 23

### 2(1,4-Dibenzylpiperazin-2-yl)-benzimidazol-4-carbonsäureamid x 2 HCl

¹H-NMR (D₆-DMSO) : δ = 2,95-3,7 (7H), 3,8-4,9 (4H), 7,1-7,55 (8H), 7,65 (2H), 7,85 (2H), 7,94 (1H), 8,7 (breit) und 12,2 (breit) ppm·

### Beispiel 24

### 2(N-Benzylpiperidin-4-yl)-1-(1-benzylpiperidin-4-ylcarbonyl)-benzimidazol-4-carbonsäureamid

¹H-NMR (D₆-DMSO) : δ = 1,7(2H), 1,8-2,0(6H), 2,1(4H), 2,5-2,7(2H), 2,8-3,0(4H), 3,5(4H), 7,2-7,5(11H), 7,7(1H), 8,6(1H), 9,5(1H) und 12,3(breit) ppm.

### Beispiel A: Hemmung des Enzyms Poly(ADP-ribose)polymerase oder PARP (EC 2.4.2.30)

Eine 96 well Mikrotiterplatte (Falcon) wird mit Histonen (Type II-AS; SIGMA H7755) beschichtet. Histone werden dazu in Carbonat-Puffer (0,05 M NaHCO₃; pH 9,4) zu einer Konzentration von 50 µg/ml gelöst. Die einzelnen Wells der Mikrotiterplatte werden über Nacht mit je 100 µl dieser Histon Lösung inkubiert. Anschließend wird die Histon Lösung entfernt und die einzelnen Wells mit 200 µl einer 1 %igen BSA (Bovine Serum Albumine) Lösung in Carbonat-Puffer für 2 Stunden bei Raumtemperatur inkubiert. Anschließend wird dreimal mit Waschpuffer (0,05 % Tween10 in PBS) gewaschen. Für die Enzymreaktion werden je Well 50 µl der Enzymreaktionslösung (5 µl Reaktions-Puffer (1M Tris-HCl pH 8,0, 100 mM MgCl₂, 10 mM DTT,) 0,5 µl PARP (c = 0,22 µg/µl), 4 µl aktivierte DNA (SIGMA D-4522, 1 mg/ml in Wasser), 40,5 µl H₂O) mit 10 µl einer Inhibitorlösung für 10 Minuten vorinkubiert. Die Enzymreaktion wird durch Zugabe von 40 µl einer Substratlösung (4 µl Reaktion-Puffer (s.o.), 8 µl NAD-Lösung (100 µM in H₂O), 28 µl H₂O) gestartet. Reaktionszeit ist 20 Minuten bei Raumtemperatur. Die Reaktion wird durch dreimaliges Waschen mit Waschpuffer (s.o.) gestoppt. Anschließend folgt eine einstündige Inkubation bei Raumtemperatur mit einem spezifischen Anti-Poly-ADP-Ribose Antikörper durchgeführt. Als Antikörper wurden ein monoklonaler anti-Poly-(ADP-ribose) Antikörpern "10H" (Biomol SA-276) verwendet.

Die Antikörper wurden in einer 1:5000 Verdünnung in Antikörper-Puffer (1%BSA in PBS; 0,05 % Tween20) eingesetzt. Nach dreimaligem Waschen mit Waschpuffer folgt eine einstündige Inkubation bei Raumtemperatur mit dem sekundärem Antikörper. Hier wurden für den monoklonalen Antikörper ein anti-Maus-IgG gekoppelt mit Peroxidase (Boehringer Mannheim) und für den Kaninchen Antikörper ein anti-Rabbit-IgG gekoppelt mit Peroxidase (SIGMA A-6154) jeweils in einer 1:10000 Verdünnung in Antikörperpuffer verwendet. Nach dreimaligem Waschen mit Waschpuffer erfolgt die Farbreaktion unter Verwendung von 100 µl/Well Farbreagenz (SIGMA, TMB-Fertigmix, T8540) für ca. 15 min. bei Raumtemperatur. Die Farbreaktion wird durch Zugabe von 100 µl 2M H₂SO₄ gestoppt. Danach wird sofort gemessen (450 gegen 620 nm; ELISA Platten Lesegerät "Easy Reader" EAR340AT, SLT-Labinstruments, Österreich. Aus den Inhibitionskurven bei verschiedenen Substratkonzentrationen kann man auf übliche Weise den Kᵢ bestimmen.

### Beispiel B: Bestimmung des Wasserlöslichkeit

Eine zu messende Verbindung wird direkt in einem festgelegten Volumen Wasser gelöst und die entstandene Lösung mit einer Natriumacetat-Lösung auf pH 5 bis 6 eingestellt, so daß die zu prüfende Konzentration des Wirkstoffs erreicht wird. Falls die Meßsubstanz nicht als wasserlösliches Salz vorliegt, wurde diese in möglichst wenig Dimethylsulfoxid gelöst und anschließend mit Wasser verdünnt (Endkonzentration an Dimethylsulfoxid ≤ 1 %), wonach auch hier der pH-Wert noch eingestellt wurde. Das erfindungsgemäße Beispiel 1 zeigte hier eine Löslichkeit > 0,5 % aufweist.

## Patentansprüche

1. Verbindungen der allgemeinen Formel Ia oder Ib 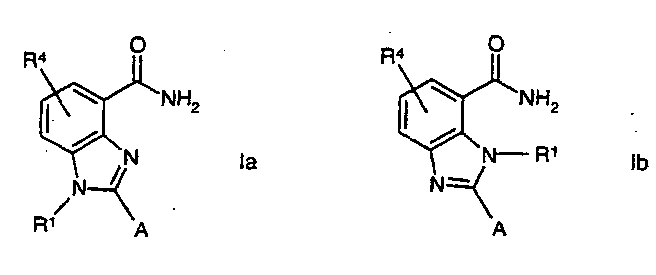 worin
R¹ Wasserstoff, verzweigtes und unverzweigtes C₁-C₆-Alkyl, wobei ein C-Atom des Alkyl-Restes noch OR⁵ (wobei R⁵ Wasserstoff oder C₁-C₄-Alkyl bedeutet), oder ein C-Atom in der Kette auch eine =O-Gruppe oder eine Gruppe NR⁸R⁹ tragen kann, wobei R⁸ und R⁹ unabhängig voneinander Wasserstoff oder C₁-C₄-Alkyl bedeuten und NR⁸R⁹ zusammen ein zyklisches Amin mit 4 bis 8 Ringatomen sein kann, wobei die C-Ketten in R⁸ bzw. R⁹ oder der durch NR⁸R⁹ gebildete Ring noch einen Rest R⁶ tragen kann, der unabhängig von R² dieselbe Bedeutung wie R² annehmen kann und
R⁴ Wasserstoff, verzweigtes und unverzweigtes C₁-C₆-Alkyl, Chlor, Brom, Fluor, Nitro, Cyano, NR⁸R⁹, NH-CO-R¹⁰, OR⁸, wobei R⁸ und R⁹ unabhängig voneinander Wasserstoff oder C₁-C₄-Alkyl bedeuten und NR⁸R⁹ zusammen ein zyklisches Amin mit 4 bis 8 Ringatomen sein kann, wobei der Ring noch einen Rest (verzweigtes und unverzweigtes C₁-C₆-Alkyl, C₃-C₇-Cycloalk-C₁-C₄-Alkyl, CO-R⁴¹, COOR⁴¹ und Phenyl) tragen kann, und R¹⁰ Wasserstoff, C₁-C₄-Alkyl oder Phenyl bedeuten kann und R⁴¹ dieselben Bedeutungen wie R²¹ annehmen kann,
A einen gesättigten oder einfach ungesättigten heterozyklischen, 4- bis 8-gliedrigen Ring, der ein oder zwei Stickstoff-Atome enthält, wobei zusätzlich noch ein Sauerstoff- oder Schwefel-Atom eingebaut sein kann, der durch die Substituenten R² und R³ substituiert ist, wobei
R² Wasserstoff, verzweigtes und unverzweigtes C₁-C₈-Alkyl, das noch mit R²³ substituiert sein kann und ein C-Atom der Kette eine =O-Gruppe tragen kann, C₃-C₇-Cycloalk-C₁-C₄-Alkyl, -CO-(NH)_{0,1}-R²¹, COOR²¹ und Phenyl bedeuten kann, wobei R²¹ Wasserstoff, verzweigtes und unverzweigtes C₁-C₆-Alkyl, C₃-C₇-Cycloalk-C₁-C₄-Alkyl, Phen-C₁-C₄-Alkyl, C₃-C₇-Cycloalkyl und Phenyl bedeuten kann und jeder Rest noch (CH₂)₀₋₂-R²³ tragen kann, und der jeweilige Phenylring seinerseits noch mit 1, 2 oder 3 der folgenden Resten substituiert sein kann: Chlor, Fluor, Brom, Jod, verzweigtes und unverzweigtes C₁-C₄-Alkyl, Nitro, CF₃, Cyano, -(CH₂)₀₋₂-NR²⁴R²⁵, NH-CO-R¹⁰, OR¹⁰, COOR¹⁰, SO₂-C₁-C₄-Alkyl, SO₂Ph, SO₂NH, NHSO₂-Cl-C₄-Alkyl, NHSO₂Ph und CF₃, wobei R²⁴ und R²⁵ unabhängig voneinander Wasserstoff oder C₁-C₄-Alkyl bedeuten und NR²⁴R²⁵ zusammen ein zyklisches Amin mit 4 bis 8 Ringatomen sein kann, wobei der Ring noch ein Rest verzweigtes und unverzweigtes C₁-C₆-Alkyl, C₃-C₇-Cycloalk-C₁-C₄-Alkyl, CO-R²², COOR²² (mit R²² gleich Wasserstoff, verzweigtes oder unverzweigtes C₁-C₆-Alkyl, C₃-C₇-Cycloalk-C₁₋C₄-Alkyl, Phen-C₁-C₄-Alkyl, C₃-C₇-Cycloalkyl und phenyl) und Phenyl tragen kann, und R¹⁰ Wasserstoff, C₁-C₄-Alkyl oder Phenyl bedeuten, und
R²³ NR²⁶R²⁷ bedeutet, wobei R²⁶ Und R²⁷ Wasserstoff, C₁-C₆-Alkyl, C₀-C₄-Alkyl-Phenyl, wobei der Phenylring noch mit bis zu 3 Resten Cl, F, Br, J, C₁-C₄-Alkyl, CF₃, CN, SO₂-C₁-C₄-Alkyl, SO₂-Phenyl, NO₂, NH₂, NHCO-C₁-C₄-Alkyl, NHCO-Phenyl, OH, O-C₁-C₄-Alkyl, O-C₁-C₄-Alkyl-Phenyl substituiert sein kann, und NR²⁶R²⁷ auch ein cyclisches Amin mit 3 bis 8 Gliedern darstellen kann, wobei zusätzlich noch ein weiteres Heteroatom wie O, N und S enthalten sein kann und der Ring noch mit einem Rest R²⁸ substituiert sein kann, wobei R²⁸ C₁-C₄-Alkyl und C₁-C₄-Alkyl-Phenyl sein kann,
R³ Wasserstoff, verzweigtes und unverzweigtes C₁-C₈-Alkyl, gegebenenfalls durch C₁-C₆-Alkyl substituiertes C₃-C₇-Cycloalk-C₁-C₄-Alkyl, gegebenenfalls durch C₁-C₆-Alkyl substituiertes C₃-C₇-Cycloalkyl, wobei ein C-Atom des Restes noch einen Phenyl-Ring tragen kann, der seinerseits noch mit 1, 2 oder 3 der folgenden Resten substituiert sein kann: Chlor, Fluor, Brom, Jod, verzweigtes und unverzweigtes C₁-C₄-Alkyl, Nitro, Cyano, (CH₂)₀₋₂-NR³²R³³, NH-CO-R¹⁰, OR¹⁰, COOR¹⁰, SO₂-C₁-C₄-Alkyl, SO₂Ph, SO₂NH₂, NHSO₂-C₁-C₄-Alkyl, NHSO₂Ph und CF₃, wobei R³² und R³³ unabhängig voneinander Wasserstoff oder C₁-C₄-Alkyl bedeuten und NR³²R³³ zusammen ein zyklisches Amin mit 4 bis 8 Ringatomen sein kann, wobei der Ring noch ein Rest verzweigtes und unverzweigtes C₁-C₆-Alkyl, C₃-C₇-Cycloalk-C₁-C₄-Alkyl, CO-R³¹, COOR³¹ und Phenyl tragen kann, und R¹⁰ Wasserstoff, C₁-C₄-Alkyl oder Phenyl bedeuten, und R³¹ die selbe Bedeutung wie R²¹ annehmen kann,
sowie ihre tautomeren Formen, möglichen enantiomeren und diastereomeren Formen, deren Prodrugs ausgewählt aus der Gruppe der Phosphate, Carbamate von Aminosäuren und Ester der allgemeinen Formel I, sowie mögliche physiologisch verträgliche Salze.

2. Verbindungen nach Anspruch 1, wobei R¹, R² und R⁴ Wasserstoff darstellen und A Piperidin, Pyrrolidin, Piperazin, Morpholin oder Homopiperazin ist und R³ an den Stickstoff von A gebunden ist.

3. Verbindungen nach einem der Ansprüche 1 oder 2, wobei A Piperdin, das mit der 4-Stellung am Benzimidazol gebunden ist und R³ Wasserstoff, C₁-C₆-Alkyl, Benzyl und Phenylethyl sein kann und in 1-Stellung am Piperidin-Ring steht, bedeuten.

4. Arzneimittel enthaltend neben den üblichen Träger und Hilfsstoffen eine Verbindung nach einem der Ansprüche 1 bis 3.

5. Verwendung von Verbindungen der Formel I nach einem der Ansprüche 1 bis 3 zur Herstellung von Arzneimitteln zur Behandlung von Krankheiten, bei denen pathologisch erhöhte Aktivitäten von PARP auftreten.

6. Verwendung von Verbindungen der Formel I nach einem der Ansprüche 1 bis 3 zur Herstellung von Arzneimitteln zur Behandlung von neurodegenerativen Krankheiten und neuronalen Schädigungen.

7. Verwendung nach Anspruch 6 zur Behandlung von solchen neurodegenerativen Krankheiten und neuronalen Schädigungen, die durch Ischämie, Trauma oder Massenblutungen ausgelöst werden.

8. Verwendung nach Anspruch 6 zur Behandlung des Schlaganfalls und des Schädel-Hirntraumas.

9. Verwendung nach Anspruch 6 zur Behandlung der Alzheimerschen Krankheit, der Parkinsonsche Krankheit und der Huntington-Krankheit.

10. Verwendung von Verbindungen der Formel I nach einem der Ansprüche 1 bis 3 zur Herstellung von Arzneimitteln zur Behandlung oder Prophylaxe von Schädigungen durch Ischämien.

11. Verwendung von Verbindungen der Formel I nach einem der Ansprüche 1 bis 3 zur Herstellung von Arzneimitteln zur Behandlung von Epilepsien, insbesondere von generalisierten epileptischen Anfällen, wie zum Beispiel Petit mal und tonisch-clonische Anfälle und partiell epileptischen Anfällen, wie Temporal Lope, und komplex-partiellen Anfällen.

12. Verwendung von Verbindungen der Formel I nach einem der Ansprüche 1 bis 3 zur Herstellung von Arzneimitteln zur Behandlung von Schädigungen der Nieren nach renalen Ischämien, Schädigungen, die durch medikamentöse Therapie verursacht werden, und zur Behandlung während und nach Nierentransplantationen.

13. Verwendung von Verbindungen der Formel I nach einem der Ansprüche 1 bis 3 zur Herstellung von Arzneimitteln zur Behandlung von Schädigungen des Herzens nach cardialen Ischämien und Schädigungen, die durch Reperfusion von verengten bzw. verschlossenen Gefäßen verursacht werden.

14. Verwendung von Verbindungen der Formel I nach einem der Ansprüche 1 bis 3 zur Herstellung von Arzneimitteln zur Behandlung von Mikroinfarkten.

15. Verwendung von Verbindungen der Formel I nach einem der Ansprüche 1 bis 3 zur Herstellung von Arzneimitteln zur Behandlung bei einer Revascularisation kritischer verengter Koronararterien.

16. Verwendung von Verbindungen der Formel I nach einem der Ansprüche 1 bis 3 zur Herstellung von Arzneimitteln zur Behandlung des akuten Myocardinfarktes und von Schädigungen während und nach dessen medikamentöser oder mechanischer Lyse.

17. Verwendung von Verbindungen der Formel I nach einem der Ansprüche 1 bis 3 zur Herstellung von Arzneimitteln zur Behandlung von Tumoren und deren Metastasierung.

18. Verwendung von Verbindungen der Formel I nach einem der Ansprüche 1 bis 3 zur Herstellung von Arzneimitteln zur Behandlung von Sepsis und des Multiorganversagens.

19. Verwendung von Verbindungen der Formel I nach einem der Ansprüche 1 bis 3 zur Herstellung von Arzneimitteln zur Behandlung von immunologischen Krankheiten wie Entzündungen und rheumatische Erkrankungen.

20. Verwendung von Verbindungen der Formel I nach einem der Ansprüche 1 bis 3 zur Herstellung von Arzneimitteln zur Behandlung von Diabetes mellitus.

## Claims

1. Compounds of the general formula Ia or Ib 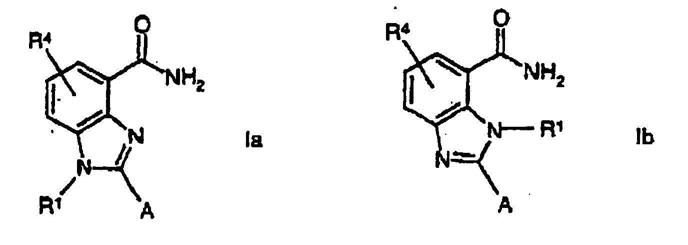 where
R¹ is hydrogen or branched or straight-chain C₁-C₆-alkyl, where one carbon atom of the alkyl radical may furthermore carry OR⁵ (where R⁵ is hydrogen or C₁-C₄-alkyl), or one carbon atom in the chain may also carry an =O group or a group NR⁸R⁹, where R⁸ and R⁹, independently of one another, are each hydrogen or C₁-C₄-alkyl and NR⁸R⁹ together may be a cyclic amine having from 4 to 8 ring atoms, where the carbon chains in R⁸ or R⁹ or the ring formed by NR⁸R⁹ may furthermore carry a radical R⁶ which, independently of R², may have the same meaning as R², and
R⁴ is hydrogen, branched or straight-chain C₁-C₆-alkyl, chlorine, bromine, fluorine, nitro, cyano, NR⁸R⁹, NH-CO-R¹⁰ or OR⁸, where R⁸ and R⁹, independently of one another, are each hydrogen or C₁-C₄-alkyl and NR⁸R⁹ together may be a cyclic amine having from 4 to 8 ring atoms, where the ring may furthermore carry a radical (branched or straight-chain C₁-C₆-alkyl, C₃-C₇-cycloalkyl-C₁-C₄-alkyl, CO-R⁴¹, COOR⁴¹ or phenyl), and R¹⁰ may be hydrogen, C₁-C₄-alkyl or phenyl and R⁴¹ may have the same meanings as R²¹,
A is a saturated or monounsaturated heterocyclic, 4- to 8-membered ring which contains one or two nitrogen atoms, it being possible for an oxygen or sulfur atom additionally to be incorporated, which is substituted by the substituents R² and R³, where
R² may be hydrogen, branched or straight-chain C₁-C₈-alkyl which may furthermore be substituted by R²³, and one carbon atom of the chain may carry an =O group, C₃-C₇-cycloalkyl-C₁-C₄-alkyl, -CO-(NH)_{0,1}-R²¹, COOR²¹ or phenyl, where R²¹ may be hydrogen, branched or straight-chain C₁-C₆-alkyl, C₃-C₇-cycloalkyl-C₁-C₄-alkyl, phenyl-C₁-C₄-alkyl, C₃-C₇-cycloalkyl or phenyl, and each radical may furthermore carry (CH₂)₀₋₂-R²³, and the respective phenyl ring in turn may furthermore be substituted by 1, 2 or 3 of the following radicals: chlorine, fluorine, bromine, iodine, branched and straight-chain C₁-C₄-alkyl, nitro, CF₃, cyano, - (CH₂)₀₋₂-NR²⁴R²⁵, NH-CO-R¹⁰, OR¹⁰, COOR¹⁰, SO₂-C₁-C₄-alkyl, SO₂Ph, SO₂NH, NHSO₂-C₁-C₄-alkyl, NHSO₂Ph and CF₃, where R²⁴ and R²⁵, independently of one another, are each hydrogen or C₁-C₄-alkyl and NR²⁴R²⁵ together may be a cyclic amine having from 4 to 8 ring atoms, where the ring may furthermore carry a radical branched or straight-chain C₁-C₆-alkyl, C₃-C₇-cycloalkyl-C₁-C₄-alkyl, CO-R²², COOR²² (where R²² is hydrogen, branched or straight-chain C₁-C₆-alkyl, C₃-C₇-cycloalkyl-C₁-C₄-alkyl, phenyl-C₁-C₄-alkyl, C₃-C₇-cycloalkyl or phenyl) or phenyl, and R¹⁰ is hydrogen, C₁-C₄-alkyl or phenyl, and
R²³ is NR²⁶R²⁷, where R²⁶ and R²⁷ are each hydrogen, C₁-C₆-alkyl or C₀-C₄-alkylphenyl, where the phenyl ring may furthermore be substituted by up to 3 radicals Cl, F, Br, I, C₁-C₄-alkyl, CF₃, CN, SO₂-C₁-C₄-alkyl, SO₂-phenyl, NO₂, NH₂, NHCO-C₁-C₄-alkyl, NHCO-phenyl, OH, O-C₁-C₄-alkyl and O-C₁-C₄-alkylphenyl, and NR²⁶R²⁷ may also be a cyclic amine having from 3 to 8 members, where a further hetero atom such as O, N and S may additionally be present, and the ring may furthermore be substituted by a radical R²⁸, where R²⁸ may be C₁-C₄-alkyl or C₁-C₄-alkylphenyl,
R³ is hydrogen, branched or straight-chain C₁-C₈-alkyl, C₃-C₇-cycloalkyl-C₁-C₄-alkyl which is optionally substituted by C₁-C₆-alkyl, or C₃-C₇-cycloalkyl which is optionally substituted by C₁-C₆-alkyl, where one carbon atom of the radical may furthermore carry a phenyl ring which in turn may also be substituted by 1, 2 or 3 of the following radicals: chlorine, fluorine, bromine, iodine, branched and straight-chain C₁-C₄-alkyl, nitro, cyano, (CH₂)₀₋₂-NR³²R³³, NH-CO-R¹⁰, OR¹⁰, COOR¹⁰, SO₂-C₁-C₄-alkyl, SO₂Ph, SO₂NH₂, NHSO₂-C₁-C₄-alkyl, NHSO₂Ph and CF₃, where R³² and R³³, independently of one another, are each hydrogen or C₁-C₄-alkyl and NR³²R³³ together may be a cyclic amine having from 4 to 8 ring atoms, where the ring may furthermore carry a radical branched or straight-chain C₁-C₆-alkyl, C₃-C₇-cycloalkyl-C₁-C₄-alkyl, CO-R³¹, COOR³¹ or phenyl, and R¹⁰ is hydrogen, C₁-C₄-alkyl or phenyl, and R³¹ may have the same meaning as R²¹,
and their tautomeric forms, possible enantiomeric and diastereoisomeric forms, their prodrugs selected from the group of the phosphates, carbamates of amino acids and esters of the general formula I, and possible physiologically tolerable salts.

2. Compounds according to claim 1, wherein R¹, R² and R⁴ are each hydrogen and A is piperidine, pyrrolidine, piperazine, morpholine or homopiperazine and R³ is bonded to the nitrogen of A.

3. Compounds according to either of claims 1 and 2, wherein A is piperidine, which is bonded at the 4-position on the benzimidazole, and R³ may be hydrogen, C₁-C₆-alkyl, benzyl or phenylethyl and is in the 1-position on the piperidine ring.

4. Medicament containing a compound according to any one of claims 1 to 3 in addition to conventional carriers and auxiliary substances.

5. Use of compounds of the formula I according to any one of claims 1 to 3 for the manufacture of medicaments for treating disorders in which pathologically increased PARP activities occur.

6. Use of compounds of the general formula I according to any one of claims 1 to 3 for the manufacture of medicaments for treating neurodegenerative disorders and neuronal damage.

7. Use according to claim 6 for treating neurodegenerative disorders and neuronal damage caused by ischaemia, trauma or massive bleeding.

8. Use according to claim 6 for treating stroke and craniocerebral trauma.

9. Use according to claim 6 for treating Alzheimer's disease, Parkinson's disease and Huntington's disease.

10. Use of compounds of the formula I according to any one of claims 1 to 3 for the manufacture of medicaments for the treatment or prophylaxis of damage caused by ischaemias.

11. Use of compounds of the formula I according to any one of claims 1 to 3 for the manufacture of medicaments for treating epilepsies, in particular generalised epileptic attacks, for example petit mal and tonoclonic attacks and partial epileptic attacks, such as temporal lobe, and complex partial attacks.

12. Use of compounds of the formula I according to any one of claims 1 to 3 for the manufacture of medicaments for treating renal damage following renal ischaemias, damage caused by drug therapy, and for treatment during and after kidney transplantations.

13. Use of compounds of the formula I according to any one of claims 1 to 3 for the manufacture of medicaments for treating cardiac damage following myocardial ischaemias and damage caused by reperfusion of narrowed or closed vessels.

14. Use of compounds of the formula I according to any one of claims 1 to 3 for the manufacture of medicaments for treating microinfarcts.

15. Use of compounds of the formula I according to any one of claims 1 to 3 for the manufacture of medicaments for treating critically narrowed coronary arteries in revascularisation.

16. Use of compounds of the formula I according to any one of claims 1 to 3 for the manufacture of medicaments for treating acute myocardial infarction and damage during and after its lysis by means of medicaments or mechanically.

17. Use of compounds of the formula I according to any one of claims 1 to 3 for the manufacture of medicaments for treating tumours and their metastasis.

18. Use of compounds of the formula I according to any one of claims 1 to 3 for the manufacture of medicaments for treating sepsis and multiorgan failure.

19. Use of compounds of the formula I according to any one of claims 1 to 3 for the manufacture of medicaments for treating immunological disorders, such as inflammations and rheumatic diseases.

20. Use of compounds of the formula I according to any one of claims 1 to 3 for the manufacture of medicaments for treating diabetes mellitus.

## Revendications

1. Composés de formule générale Ia ou Ib 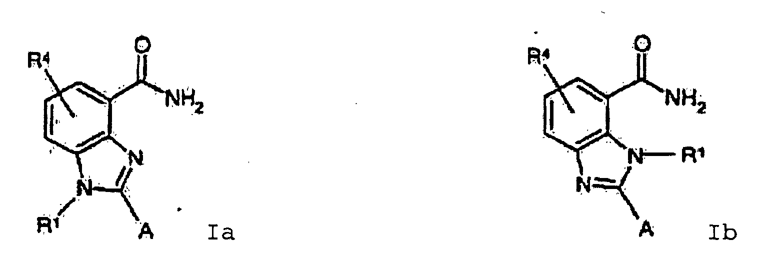 où
R₁ est un hydrogène, un C₁-C₆-alkyle ramifié ou non ramifié, un atome de carbone du radical alkyle pouvant encore porter un OR⁵ (R⁵ signifiant un hydrogène ou un C₁-C₄-alkyle), ou un atome de carbone de la chaîne pouvant aussi porter un groupe =O ou un groupe NR⁸R⁹, R⁸ et R⁹ signifiant indépendamment l'un de l'autre hydrogène ou C₁-C₄-alkyle, et NR⁸R⁹ ensemble pouvant être une amine cyclique avec 4 à 8 atomes dans le cycle, les chaînes de carbones en R⁸ et R⁹ ou le cycle formé par NR⁸R⁹ pouvant encore porter un radical R⁶ qui peut prendre indépendamment de R² la même signification que R², et
R⁴ est un hydrogène, un C₁-C₆-alkyle ramifié ou non-ramifié, un chlore, un brome, un fluor, un nitro, un cyano, un NR⁸R⁹, un NH-CO-R¹⁰, un OR⁸, R⁸ et R⁹ signifiant indépendamment l'un de l'autre hydrogène ou C₁-C₄-alkyle et NR⁸R⁹ ensemble pouvant être une amine cyclique avec 4 à 8 atomes dans le cycle, le cycle pouvant porter encore un radical (C₁-C₆-alkyle ramifié ou non ramifié, C₃-C₇-cycloalco-C₁-C₄-alkyle, CO-R⁴¹, COOR⁴¹ et phényle), et R¹⁰ pouvant signifier hydrogène, C₁-C₄-alkyle, ou phényle et R⁴¹ pouvant prendre les même significations que R²¹,
A est un hétérocycle saturé ou simplement insaturé, cycle de 4 à 8 atomes qui contient un ou deux atomes d'azote, pouvant de plus être constitué d'encore un atome d'oxygène ou de soufre, qui est substitué par les substituants R² et R³,
R² pouvant signifier hydrogène, C₁-C₈-alkyle ramifié ou non ramifié qui peut encore être substitué par R²³ et un atome de carbone de la chaîne pouvant encore porter un groupe =O, C₃-C₇-cycloalco-C₁-C₄-alkyle, -CO- (NH)_{0,1}-R²¹, COOR²¹ et phényle, R²¹ pouvant signifier hydrogène, C₁-C₆-alkyle ramifié ou non ramifié, C₃-C₇-cycloalco-C₁-C₄-alkyle, phéno-C₁-C₄-alkyle, C₃-C₇-cycloalkyle et phényle et chaque radical pouvant quant à lui encore porter un (CH₂)₀₋₂-R²³, et le cycle du phényle considéré pouvant être substitué par 1, 2, ou 3 des radicaux suivants : chlore, fluor, brome, iode, C₁-C₄-alkyle ramifié et non ramifié, nitro, CF₃, cyano, -(CH₂)₀₋₂-NR²⁴R²⁵, NH-CO-R¹⁰, OR¹⁰, COOR¹⁰, SO₂-C₁-C₄-alkyle, SO₂Ph, SO₂NH, NHSO₂-C₁-C₄-Alkyle, NHSO₂Ph et CF₃, R²⁴ et R²⁵ signifiant indépendamment l'un de l'autre hydrogène ou C₁-C₄-alkyle et NR²⁴R²⁵ ensemble pouvant être une amine cyclique avec 4 à 8 atomes dans le cycle, le cycle pouvant encore porter un radical C₁-C₆-alkyle ramifié ou non ramifié, C₃-C₇-cycloalco-C₁-C₄-alkyle, CO-R²², COOR²² (avec R²² également un hydrogène, un C₁-C₆-alkyle ramifié ou non ramifié, un C₃-C₇-cycloalco-C₁-C₄-alkyle, un phéno-C₁-C₄-alkyle, un C₃-C₇-cycloalkyle et un phényle) et un phényle, et R¹⁰ signifiant hydrogène, C₁-C₄-alkyle ou phényle, et
R²³ signifiant NR²⁶R²⁷, R²⁶ et R²⁷ étant un hydrogène, un C₁-C₆-alkyle, un C₀-C₄-alkyl-phényle, le cycle du phényle pouvant encore être substitué par jusqu'à trois radicaux Cl, F, Br, I, C₁-C₄-alkyle, CF₃, CN, SO₂-C₁-C₄ -alkyle, SO₂-phényle, NO₂, NH₂, NHCO-C₁-C₉-alkyle, NHCO-phényle, OH, O-C₁-C₄-alkyle, O-C₁-C₄-alkyl-phényle, et NR²⁶R²⁷ pouvant présenter aussi une amine cyclique avec 3 à 8 chaînons, un hétéroatome supplémentaire comme O, N et S pouvant de plus être contenu et le cycle pouvant encore être substitué par un radical R²⁸, R²⁸ pouvant être un C₁-C₄-alkyle et un C₁-C₄-alkyl-phényl,
R³ est un hydrogène, un C₁-C₈-alkyle ramifié et non ramifié, un C₃-C₇-cycloalco-C₁-C₄-alkyle substitué le cas échéant par un C₁-C₆-alkyle, un C₃-C₇-cycloalkyle substitué le cas échéant par un C₁-C₆-alkyle, l'un des atomes de carbone du radical pouvant encore porter un cycle de phényle, qui de son côté peut être substitué par un, deux ou trois des radicaux suivants : chlore, fluor, brome, iode, C₁-C₄-alkyle ramifié et non ramifié, nitro, cyano, (CH₂)₀₋₂-NR³²R³³, NH-CO-R¹⁰, OR¹⁰, COOR¹⁰, SO₂-C₁-C₄-alkyle, SO₂Ph, SO₂NH₂, NHSO₂-C₁-C₄-alkyle, NHSO₂Ph et CF₃, R³² et R³³ signifiant indépendamment hydrogène ou C₁-C₄-alkyle et NR³²R³³ pouvant être une amine cyclique avec 4 à 8 atomes dans le cycle, le cycle pouvant encore porter un radical C₁-C₆-alkyle ramifié et non ramifié, C₃-C₇-cycloalco-C₁-C₄-alkyle, CO-R³¹, COOR³¹ et phényle, et R¹⁰ signifiant hydrogène, C₁-C₄-alkyle ou phényle, et R³¹ pouvant prendre la même signification que R²¹,
ainsi que leurs formes tautomères, leurs éventuelles formes énantiomères et diastéréoisomères, leurs promédicaments choisis dans le groupe des phosphates, des carbamates d'acides aminés et des esters de la formule générale I, ainsi que leurs éventuels sels sans effet secondaire physiologique.

2. Composés selon la revendication 1, dans lesquels R¹, R² et R⁴ représentent un hydrogène et A la pipéridine, la pyrrolidine, la pipérazine, la morpholine ou l'homopipérazine, et R³ est lié à l'azote de A.

3. Composés selon l'une des revendications 1 ou 2, dans lesquels A signifie la pipéridine qui est reliée à la position 4 au benzimidazole et R³ peut être un hydrogène, un C₁-C₆-alkyle, un benzyle ou un phényléthyle et se situe en position 1 sur le cycle de pipéridine.

4. Médicaments contenant à côté des porteurs et auxiliaires habituels un composé selon l'une des revendications 1 à 3.

5. Utilisation de composés de formule générale I selon l'une des revendications 1 à 3 en vue de la préparation de médicaments pour le traitement de maladies dans lesquelles apparaissent des activités pathologiques élevées de PARP.

6. Utilisation de composés de formule 1 selon l'une des revendications 1 à 3 en vue de la préparation de médicaments pour le traitement de maladies neurodégénératives et d'affections neuronales.

7. Utilisation selon la revendication 6 pour le traitement de telles maladies neurodégénératives et affections neuronales qui sont déclenchées par ischémie, traumatisme ou hémorragie massive.

8. Utilisation selon la revendication 6 pour le traitement des attaques cérébrales et traumatismes crâniens.

9. Utilisation selon la revendication 6 pour le traitement de la maladie d'Alzheimer, de la maladie de Parkinson et de la maladie de Huntington.

10. Utilisation de composés de formule I selon l'une des revendications 1 à 3 en vue de la préparation de médicaments pour le traitement ou la prophylaxie d'affections par ischémie.

11. Utilisation de composés de formule I selon l'une des revendications 1 à 3 en vue de la préparation de médicaments pour le traitement de l'épilepsie, en particulier pour les crises d'épilepsie généralisées, comme le petit mal et les crises tonico-cloniques, et les crises d'épilepsie partielles, comme les accès psychomoteurs et les crises partielles complexes.

12. Utilisation de composés de formule I selon l'une des revendications 1 à 3 en vue de la préparation de médicaments pour le traitement d'affections des reins après des ischémies rénales, des affections qui sont causées par une thérapie médicamenteuse, et pour le traitement pendant et après transplantation du rein.

13. Utilisation de composés de formule I selon l'une des revendications 1 à 3 en vue de la préparation de médicaments pour le traitement d'affections du coeur après des ischémies cardiaques et des affections qui sont causées par des reperfusions de vaisseaux rétrécis ou bouchés.

14. Utilisation de composés de formule I selon l'une des revendications 1 à 3 en vue de la préparation de médicaments pour le traitement de microinfarctus.

15. Utilisation de composés de formule I selon l'une des revendications 1 à 3 en vue de la préparation de médicaments pour le traitement dans une revascularisation d'artères coronaires rétrécies de façon critique.

16. Utilisation de composés de formule I selon l'une des revendications 1 à 3 en vue de la préparation de médicaments pour le traitement de l'infarctus du myocarde aigu et d'affections pendant et après le traitement médicamenteux ou mécanique de celui-ci.

17. Utilisation de composés de formule I selon l'une des revendications 1 à 3 en vue de la préparation de médicaments pour le traitement de tumeurs et de leurs métastases.

18. Utilisation de composés de formule I selon l'une des revendications 1 à 3 en vue de la préparation de médicaments pour le traitement de septicémies et de défaillances multiorganes.

19. Utilisation de composés de formule I selon l'une des revendications 1 à 3 en vue de la préparation de médicaments pour le traitement de maladies immunologiques comme des inflammations et des affections rhumatismales.

20. Utilisation de composés de formule I selon l'une des revendications 1 à 3 en vue de la préparation de médicaments pour le traitement du diabète sucré.
